# FASCICULE DE BREVET EUROPEEN

(11) **EP 1 517 919 B1**
(45) Date de publication et mention de la délivrance du brevet: **11.03.2009**
(21) Numéro de dépôt: 03761669.5
(22) Date de dépôt: 30.06.2003
(51) Int. Cl.: C07K 14/47, C12N 15/12, G01N 33/58, A61K 51/08, A61P 7/02, A61P 29/00, C07D 401/06, C07D 401/12

(54) **PEPTIDES MARQUES AYANT UNE AFFINITE POUR UN PHOSPHOLIPIDE ET UTILISATIONS**
MARKIERTE PEPTIDE MIT EINER AFFINITÄT FÜR EIN PHOSPHOLIPID UND DEREN VERWENDUNGEN
MARKED PEPTIDES HAVING AFFINITY FOR A PHOSPHOLIPID AND USES

(30) Priorité: 01.07.2002 FR 0208204
(43) Date de publication de la demande: 30.03.2005
(73) Titulaire: COMMISSARIAT A L'ENERGIE ATOMIQUE, 75752 Paris Cédex 15 (FR); Université Pierre et Marie Curie (Paris VI), 75252 Paris Cédex 05 (FR)
(72) Inventeur: SANSON, Alain, F-91940 GOMETZ LE CHATEL (FR); OCHSENBEIN, Françoise, F-91190 Gif Sur Yvette (FR); DOLLE, Frédéric, F-91940 GOMETZ LE CHATEL (FR)
(74) Mandataire: Poulin, Gérard
(86) Numéro de dépôt international: PCT/FR2003/002027
(87) Numéro de publication internationale: WO 2004/003016

(56) Documents cités:
- EP-A- 0 293 567
- WO-A-00/20453
- WO-A-92/19279
- US-A- 4 735 792
- MONTAVILLE PIERRE ET AL: "A new consensus sequence for phosphatidylserine recognition by annexins." THE JOURNAL OF BIOLOGICAL CHEMISTRY. UNITED STATES 5 JUL 2002, vol. 277, no. 27, 5 juillet 2002 (2002-07-05), pages 24684-24693, XP002268388 ISSN: 0021-9258
- C-Y SHIUE: "Synthesis of 18F-labelled N-(p-[18F]fluorophenyl)maleimide and its derivatives for labelling monoclonal antibody with 18F" JOURNAL OF LABELLED COMPOUNDS AND RADIOPHARMACEUTICALS, SUSSEX, GB, vol. 26, 1989, pages 287-289, XP002091356 ISSN: 0362-4803 cité dans la demande

## Description

### DOMAINE TECHNIQUE

La présente invention se rapporte à une famille de peptides marqués par le Fluor-18 qui ont des affinités améliorées pour les phospholipides, et à leurs utilisations.

De manière générale, les peptides de la présente invention sont utiles pour la reconnaissance spécifique de molécules lipidiques. Ils sont utilisables pour l'ingénierie et la création de composés de reconnaissance et de séquestration de lipides notamment de lipides chargés négativement, tels que les phosphatidylsérines, les acides phosphatidiques et lyso-phosphatidiques, les phosphatidylglycérols, les cardiolipines et les sphyngosines-1-phosphates.

Les lipides précités jouent un rôle important notamment dans la signalisation cellulaire et peuvent être présents à la surface externe des membranes des cellules et/ou circuler dans le milieu sanguin à la suite d'événements pathologiques très divers.

Divers événements cellulaires aboutissent à l'apparition de lipides chargés négativement et notamment de phosphatidylsérines (PS) à la surface externe des cellules, ces événements peuvent résulter soit d'une altération fortuite ou pathologique de la cellule, soit d'un événement cellulaire programmé telle que la mort cellulaire ou apoptose. L'apparition de PS à la surface externe des cellules constitue donc un "message primaire" important témoignant de l'existence d'un dysfonctionnement. Dans le cas du processus de coagulation sanguine, le mécanisme est bien décrit : l'altération des cellules endothéliales des vaisseaux sanguins, soit pour des raisons accidentelles, soit pour des raisons pathologiques plus complexes, provoque l'apparition de ce message PS à la surface externe des cellules en contact avec le milieu sanguin. Ce message est immédiatement reconnu par certaines protéines circulantes qui déclenchent alors une cascade d'événements aboutissant au phénomène de coagulation sanguine bien connu.

L'invention tire profit de la propriété des peptides marqués qu'elle fournit, de se lier, en présence ou non de calcium, aux lipides et notamment à ceux chargés négativement, pour la mise au point de composés utilisables comme outils de recherche et de diagnostic dans le domaine de la reconnaissance des effecteurs lipidiques et de la détection de l'apoptose, des troubles de la coagulation sanguine, du choc septique et des pathologies inflammatoires aiguës en particulier.

Les peptides marqués de l'invention sont couplés à un halogène radioactif, émetteur à positons, qui est le fluor ¹⁸F. Avec ces peptides marqués, il est donc possible par exemple de détecter des cellules apoptotiques ou de reconnaître des microdomaines membranaires chargés négativement.

Ils peuvent être utilisés pour une détection "*in vitro*" de pathologies impliquant l'apparition de centres exposant des lipides chargés négativement à la surface de cellules et/ou la libération dans le sang de microvésicules.

Les peptides marqués de la présente invention peuvent également être utilisés pour la détection in vivo et l'imagerie des foyers apoptotiques, de zones thrombotiques, et de manière générale de centres exposant des lipides chargés négativement à la surface de cellules et/ou la libération dans le sang de microvésicules, par exemple au moyen d'images scintigraphiques, acquises en tomographie par émission de positons (PET pour « Positron Emission Tomography »).

D'autres applications apparaîtront encore à l'homme du métier à la lecture de la description qui suit.

### ETAT DE LA TECHNIQUE

Une famille de protéines, appelées annexines, ont été décrites dans l'art antérieur comme présentant un ancrage fonctionnel réversible à la membrane cellulaire, régulé par la concentration en calcium et la présence de phospholipides anioniques. Les annexines constituent une famille de protéines exprimées dans des tissus très divers, aussi bien chez les animaux que chez les plantes. Il semble qu'elles ne sont ni exprimées chez la bactérie, ni chez la levure.

La structure des annexines comporte quatre domaines d'environ 70 acides aminés, ou résidus, très moyennement homologues en séquence mais de topologie quasiment identique.

Dans le document WO 92/19279, J. TAIT décrit des conjugués ayant une affinité pour des phospholipides. Il décrit en particulier l'utilisation d'une annexine, en particulier de l'annexine V, pour fabriquer un conjugué actif utilisable en tant qu'agent thrombolytique.

Malheureusement, le composé décrit dans ce document et préparé à partir de l'annexine entière par un procédé de recombinaison génétique, possède de nombreux inconvénients qui sont notamment un rendement faible, un coût de fabrication élevé. Les inconvénients majeurs sont surtout l'obtention d'un conjugué fragile du fait de sa topologie complexe conduisant à un dépliement irréversible. En outre, ces molécules présentent une toxicité majeure pour le rein et le coeur. L'article de Montaville et al. 2002 (J.Biol. Chem., vol. 277, pages 24684-24693) décrit une séquence consensus grandement conservée parmi les annexines et responsable de leur liaison aux membranes exposant des phospholipides.

Les présents inventeurs ont décrit dans la demande WO-A-00/20453 une première famille de peptides palliant les inconvénients précités et présentant une affinité pour les phospholipides et une stabilité améliorées.

Par ailleurs, on sait que pour une utilisation dans la recherche ou le diagnostic les macromolécules, telles que les protéines ou encore les peptides peuvent être couplés à une molécule de marquage permettant leur détection, cette molécule de marquage peut être, par exemple, une molécule fluorescente, des particules d'or, un composé paramagnétique ou une molécule portant un radioélément.

Les protéines ont été marquées de manière radioactives par des radioisotopes, de l'iode et divers radioisotopes de métaux, tels que le technétium, l'indium et le gallium. Plus récemment, les protéines ont été marquées avec du fluor-18.

Par exemple, les peptides couplées à des radioéléments, tels que le fluor, permettent une détection « in vivo » de la localisation des zones thrombotiques lors d'accidents vasculaires de toutes sortes, en particulier des foyers apoptatiques et inflammatoires, en utilisant des systèmes d'imagerie.

Ainsi, les atomes radioactifs émetteurs de positons à durée de vie courte et notamment le ¹⁸F peuvent, en particulier, être détectés par les appareils de tomographie par émission de positons (TEP) (PET ou « Positon Emission Tomography »).

Le marquage radioactif par le fluor-18, pose, notamment du fait de la très courte période du fluor-18 (voisine de 109,8 minutes) des problèmes spécifiques qui font que le marquage par le fluor-18 est fondamentalement différent de celui avec les autres halogènes, tels que l'iode.

Le couplage précité peut être réalisé par toutes les techniques classiques de chimie organique connues de l'homme du métier, et par la synthèse de marqueurs de protéines et de peptides portant un ou plusieurs atomes radioactifs à durée de vie courte en particulier le ¹⁸F. Ce marqueur est généralement constitué, d'une part, d'une partie capable de recevoir, par exemple, un atome de ¹⁸F et, d'autre part, d'une partie comportant une fonction classique quelconque de liaison à la macromolécule, par exemple, à la protéine.

Ces marqueurs doivent répondre à l'exigence d'une synthèse rapide et facile, car du fait de la courte durée de vie des radio isotopes tels que le ¹⁸F, la durée de synthèse ne doit pas généralement excéder quelques heures.

En outre, cette synthèse, du fait de la haute radioactivité des composés mis en oeuvre, doit pouvoir être réalisée par des moyens robotisés.

Ainsi, les procédés pour le marquage de protéines ou de peptides avec le fluor-18 font-ils appel à des marqueurs encore appelés « conjugués » ou « synthon » marqués, qui sont classés en trois familles principales, selon qu'ils réagissent avec les groupes amines, les groupes sulfhydryles, ou les groupes carbohydrates des macromolécules, tels que les protéines et peptides.

Parmi les composés ou conjugués réagissant avec les groupes amino, on peut citer les imidates, tels que le 3-[¹⁸F]fluoro-5-nïtrobenzoimidate, qui réagissent, par exemple, avec le groupe ε-NH₂ de la lysine pour se lier à une protéine ; les esters activés, tels que le N-succinimidyl- [¹⁸F]fluorobenzoate; les acides carboxyliques, tels que l'acide N-(4-[¹⁸F]fluorobenzoïque) ; les aldéhydes, telles que la 4- [¹⁸F] pentafluorobenzaldéhyde et les isothiocyanates, tels que le 4-([¹⁸F]fluorométhyl phénylisothiocyanate).

Les halogénures activés, tels que le bromure de (4-[¹⁸F]fluorophénacyle), réagissent avec les groupes amino, tels que le groupe ε-NH₂ de la lysine ou le groupe -SH de la cystéine.

Les amines, telles que la 1-(4-([¹⁸F]fluorométhyl)benzoyl)-aminobutane-4-amine réagissent avec les groupes CO₂H, par exemple de l'acide glutamique ou de l'acide aspartique ou avec les groupes CHO des glycoprotéines.

Les nitrènes avec des centres actifs photochimiques, tels que le fluorure [¹⁸F] d'azidophénacyle réagissent aussi avec les groupes amino, par exemple le groupe ε-NH₂ de la lysine.

Le procédé le plus efficace et le plus décrit pour marquer les protéines et peptides est celui qui met en oeuvre des acides activés, mais c'est aussi le procédé qui présente la non-spécificité la plus grande car tous les sites nucléophiles des aminoacides des protéines ou peptides vont réagir avec le marqueur, conjugué, ou synthon marqué.

Deux procédés plus spécifiques pour marquer les peptides et les nucléotides présentent une bonne spécificité vis-à-vis des atomes de soufre, par exemple de la cystéine pour les peptides et d'une fonction phosphoro-thioate pour les nucléotides.

Il s'agit, tout d'abord, des procédés mettant en oeuvre des « synthons » haloacétamides, qui, bien que satisfaisants, présentent l'inconvénient d'être très lents et donc peu adaptés au ¹⁸F, du fait de la période de celui-ci.

Il s'agit ensuite des procédés mettant en jeu des maléimides activés, qui peuvent se fixer sur les groupes SH avec une très bonne spécificité car la réaction est très lente vis-à-vis, par exemple des sites ε-NH₂ de la lysine.

Le schéma de la réaction impliquant le groupe maléimido est le suivant, dans le cas d'une protéine : 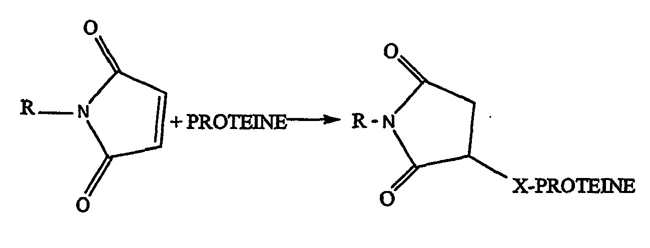 dans laquelle X représente -S-.

Pour tout marquage, quel qu'en soit le type, les molécules comprenant un radical maléimide sont, actuellement considérées comme étant les meilleures, pour ce qui est de leur réactivité avec les macromolécules, telles que les peptides ou les protéines.

Le document de SHIUE C.-Y. et al., J. Label Compounds Radiopharm 26 : 278-280 (1988), décrit les composés :

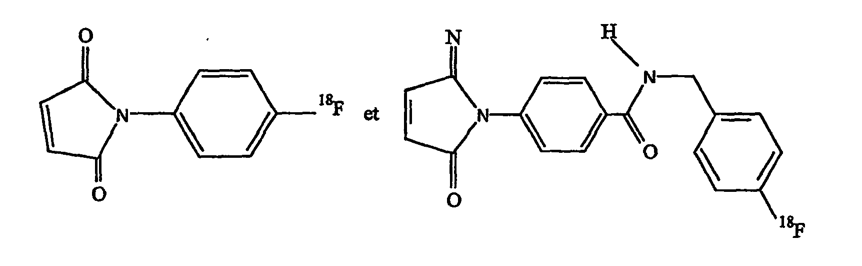

Le premier de ces composés n'est pas facile à marquer avec du fluor-18 à haute activité spécifique.

En effet, seul le fluor F₂ permettrait un marquage facile de « type iode » et il se trouve précisément que F₂ est généralement un produit à basse activité spécifique.

En particulier, le F₂ ne convient pas à la fabrication de composés dits « radiotraceurs » qui sont, préférentiellement, visés selon l'invention, tout simplement parce que la masse injectée de molécule marquée devient importante et que, alors, le principe de base régissant ce « traceur », à savoir l'occupation extrêmement faible (par exemple, inférieure à 5 %) des sites récepteurs, n'est pas respecté.

En outre, la synthèse du premier de ces composés est difficile, elle est, en effet, réalisée en quatre étapes nécessitant une durée importante avec des rendements très faibles, et des transformations chimiques relativement complexes. Ce procédé n'est donc pas susceptible d'être aisément automatisé.

Le second des composés cités dans le document de SHIUE et al. comporte une chaîne amide qui n'est pas chimiquement très solide et qui est facilement clivée, rompue, in vivo.

Sa mise en oeuvre pour des applications de diagnostic n'est donc pas envisageable. En outre, la synthèse de ce second composé comprend trois étapes et le rendement final est faible, voisin, par exemple, de 10% ("EOB" "End of Bombardment" en anglais, c'est-à-dire en fin d'irradiation).

Le document US-A-4 735 792 est relatif à des molécules de formule : 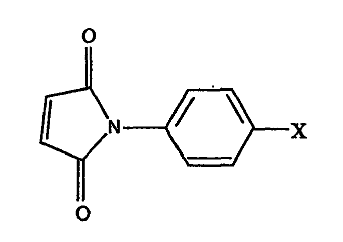 dans laquelle X est un halogène radioactif choisi parmi le brome-75, le brome-76, le brome -82, l'iode-123, l'iode-125, l'iode-131 et le fluor-18.

Toutefois, seule la molécule marquée à l'iode-125 est effectivement préparée.

La préparation d'une molécule marquée au fluor-18 n'est ni mentionnée, ni évoquée, et les remarques déjà effectuées ci-dessus, en ce qui concerne le premier composé du document de SHIUE et al., s'appliquent aussi dans le cadre du document US-A-4 735 792.

L'homme du métier, à la lecture de ce document, ne possède aucune information lui permettant de préparer spécifiquement un composé marqué au fluor-18 et s'il envisage de le faire, il mettrait en oeuvre du F₂ et aboutirait ainsi à un composé de faible activité spécifique, inutilisable en imagerie « PET ».

On peut en outre, considérer que la chimie mise en oeuvre pour fabriquer le composé fluoré du document US-A-4 735 792 est une chimie complexe et longue.

### EXPOSE DE L'INVENTION

La présente invention a précisément pour but de fournir une nouvelle famille de peptides, marqués par un halogène radioactif qui est fluor ¹⁸F grâce à un nouveau composé de marquage, le peptide ayant une affinité pour les lipides, en particulier pour les phospholipides, plus spécifique et encore améliorée par rapport aux produits de l'art antérieur ; et le composé de marquage ayant, entre autres, une forte réactivité, une grande sélectivité en particulier vis-à-vis des atomes de soufre tels que ceux des fonctions thiols des cystéines, et une bonne activité spécifique et ledit composé de marquage pouvant en outre être fabriqué par un procédé simple, fiable, facilement automatisable, rapide et de courte durée.

Les peptides de l'invention présentent en outre les avantages d'être plus stables chimiquement que les composés de l'art antérieur et de pouvoir être fabriqués de manière reproductible, avec un rendement élevé et un coût de fabrication très réduit par rapport aux composés de l'art antérieur.

Le Fluor-18 (¹⁸F) est un émetteur de positons qui permet une détection au moyen des peptides marqués de la présente invention de lipides chargés négativement dans toute zone du corps par des caméras à positons (PET). Ce couplage des peptides de la présente invention, au ¹⁸F, permet par exemple de détecter avec une résolution meilleure que le millimètre, la présence de cellules présentant la phosphatidylsérine (PS), présente à la surface externe des cellules impliquées dans des processus physiopathologiques comme la mort cellulaire programmée, l'apoptose, la coagulation du sang, la réaction inflammatoire *in vivo* chez tout être vivant. Elle permet également une telle détection in vitro dans des tests de laboratoire.

Ces peptides marqués de la présente invention permettent aussi de quantifier de manière précise par exemple le nombre de cellules présentant la phosphatidyle serine.

Les peptides de la présente invention sont tels que définis à l'une quelconque des revendications 1 à 17. La présente invention décrit également des peptides comprenant la séquence peptidique (PI) suivante : dans laquelle J, Z, U, X et B représentent des acides aminés tels que :
- les acides aminés J sont choisis indépendamment les uns des autres parmi les acides aminés naturels, ou des dérivés de ceux-ci, de telle manière qu'au moins 50% d'entre eux sont des résidus polaires choisis parmi Arg, Asn, Asp, Cys, Gln, Glu, Gly, His, Lys, Orn, Pro, Ser, Thr et Tyr,
- les acides aminés U sont choisis parmi Ala, Cys, Gly, Ile, Leu, Met, Phe, Trp, Tyr et Val,
- l'acide aminé X¹⁸ est choisi indépendamment des autres acides aminés de la séquence parmi Ala, Asn, Cys, Gln, Gly, His, Ile, Leu, Met, Phe, Ser, Thr, Trp, Tyr et Val,
- l'acide aminé B³⁷ est choisi indépendamment des autres acides aminés de la séquence parmi Arg, Ala, Cys, Gly, Ile, Leu, Met, Phe, Trp, Tyr et Val,
- l'acide aminé Z⁷ est choisi indépendamment des autres acides aminés de la séquence parmi Asp et Glu,
- les acides aminés Z⁵⁹ et Z⁶⁵ sont choisis indépendamment parmi Glu, Asp, Lys et Arg,
les exposants des J, Z, U, X et B représentant la position de ces acides aminés dans ladite séquence.

Selon l'invention, ces peptides de la présente invention, tels qu'ils sont définis ci-dessus, sont marqués directement ou indirectement avec un composé de marquage de la présente invention de formule générale (CI) suivante : 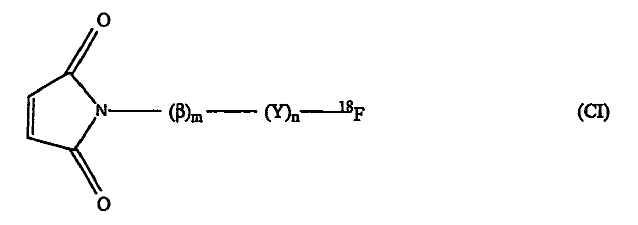 dans laquelle :
- m représente un nombre entier de 0 à 10, tel que 0, 1, 2, 3, 4, 5, 6, 7, 8, 9 ou 10 ;
- n représente un nombre entier de 0 à 10, tel que 0, 1, 2, 3, 4, 5, 6, 7, 8, 9 ou 10 ;
- Y représente un groupe choisi parmi les groupes alkyle, les groupes hétérocycliques monocycliques ou bicycliques choisis parmi les groupes imidazolyle, pyrazolyle, benzimidazolyle, pyridinyle, piridazinyle, pyrimidinyle, pyrazinyle, triazinyle, quinolinyle, isoquinolinyle, cinnolinyle, quinazolinyle, quinoxalinyle, purinyle, Y pouvant être, éventuellement, substitué par un ou plusieurs substituants, chacun de ces substituants étant indépendamment choisi parmi l'hydrogène, les halogènes (non radioactifs), les groupes phényle, alkyle en C₁₋₆, alcoxy en C₁₋₆, aryloxy, amino, mono- ou di (alkyle en C₁₋₆)amino, mono- ou di(aryl)amino, thio, alkyle en C₁₋₆-thio, arylthio, formyle, alkyle en C₁₋₆-carbonyle, arylcarbonyle, carbonyle, alcoxy en C₁₋₆-carbonyle, aryloxycarbonyle, alkyle en C₁₋₆-aminocarbonyle, arylaminocarbonyle, trifluorométhyle ;
   - β représente un radical de formule :

      (γ)ₐ-((CR₁R₂)_{b}-(V)_{c})_{d}-((CR₃R₄)ₑ-(W)_{f})_{g}-

      dans laquelle :
   - a, b, c, d, e, f, g représentent chacun indépendamment un nombre entier de 0 à 10, tel que 0, 1, 2, 3, 4, 5, 6, 7, 8, 9 ;
   - y, V et W représentent chacun indépendamment -NR-₁, -O-, -S-, 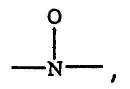 éthynyle, -CR₁=CR₂-, - (C=O)-, - (C=S) -, -C (=NR₁)-, -C=0) 0-, -(C=S) S - , -C (=NR₁) NR₂-, -CR₁R₂-, -CR₁R₂-, CR₁NR₂R₃-, où R₁, R₂, R₃ et R₄ sont chacun indépendamment choisis parmi l'hydrogène, les halogènes, les groupes phényle, alkyle en C₁₋₆, alcoxy en C₁₋₆, aryloxy, amino, mono- ou di(alkyle en C₁₋₆)amino, mono- ou di(aryl)amino, thio, alkyle en C₁₋₆-thio, arylthio, formyle, alkyle en C₁₋₆-carbonyle, arylcarbonyle, carbonyle alcoxy en C₁₋₆-carbonyle, aryloxycarbonyle, alkyle en C₁₋₆-aminocarbonyle, arylaminocarbonyle, trifluorométhyle.

Généralement, dans la présente description, halogène signifie fluor, chlore, brome ou iode. C₁₋₆ alkyle correspond aux radicaux hydrocarbonés saturés à chaînes linéaires et ramifiées ayant de 1 à 6 atomes de carbone, tels que méthyle, éthyle, propyle, butyle, pentyle et hexyle.

Le rattachement et la substitution des hétérocycles, groupe aryle, etc., peut se faire en une position quelconque.

De même, le rattachement du ¹⁸F sur Y ou β peut se faire en une position quelconque, en particulier sur une position quelconque sur un hétérocycle.

Les composés selon l'invention se distinguent fondamentalement des composés de l'art antérieur, du fait de leur structure spécifique dans laquelle la partie portant l'atome de fluor-18 est constituée, selon l'invention, par un groupe Y spécifique qui est notamment un groupe pyridinyle ; la partie de liaison, de couplage au peptide, est constituée, selon l'invention par une fonction spécifique, à savoir une fonction maléimido ; et, enfin, la partie de liaison au peptide et la partie portant l'atome de fluor-18 sont reliées selon l'invention par une chaîne ou bras espaceur également spécifique, par exemple de type alkyle (généralement de 2 à 6C), éther d'alkyle, éthers de phénylalkyle, alcényle, qui ne sont pas fragiles et ne sont pas susceptibles de ruptures « in vivo ».

Par marquage direct, on entend un couplage directe, sans intermédiaire, tel qu'un bras espaceur, du composé de marquage (CI) avec le peptide de la présente invention, par exemple grâce à une fonction -SH libre du peptide défini ci-dessus, il peut s'agir notamment de la fonction thiol d'une cystéines du peptide.

Ce couplage du composé de marquage (CI) avec le peptide peut se faire soit sur la séquence définie à la revendication 1 par exemple au niveau de résidus cystéines localisés à la surface de la protéine, mais de façon non gênante pour les fonctions de liaison du calcium et des phospholipides, soit sur une partie du peptide autre que celle de ladite séquence. Le couplage se fait par la fonction maléimide du composé (CI).

Plus précisément, ledit couplage est réalisé par réaction de la double liaison du groupe maléimido du composé selon l'invention avec spécifiquement une fonction -SH(thiol) d'une cystéine faisant partie du peptide.

C'est là un des avantages liés à la structure spécifique des composés selon l'invention que de permettre un marquage spécifique, voire exclusif, des cystéines, alors que la plupart des autres « synthons » ne permettent qu'un marquage non-spécifique des lysines et des cystéines.

Le marquage sélectif, voire exclusif, des cystéines et dû à la présence dans la molécule de marquage de l'invention d'une fonction « dédiée », à savoir la fonction maléimido, qui est une fonction dédiée pour la chemio-sélectivité envers les thiols des cystéines.

Par marquage indirect, on entend l'utilisation d'un bras espaceur lié d'une part au composé de marquage, et d'autre part au peptide tel qu'il est défini ci-dessus. Ce bras espaceur peut avoir pour fonction d'éloigner le marqueur du peptide afin qu'aucune gêne stérique n'empêche le peptide de reconnaître sa cible (lipide chargé négativement). Ce bras espaceur peut être de nature organique, par exemple un alkyle muni d'un groupe thiol, ou une séquence peptidique comprenant une cystéine par exemple -(Gly)ₙ-Cys où n est égal ou supérieur à 1.

Il est évident que le couplage du composé de marquage avec le peptide conformément à la présente invention sera en tout état de cause tel qu'il n'inhibe pas ou de manière peu gênante l'activité de reconnaissance spécifique des lipides chargés négativement par le peptide de la présente invention.

La séquence peptidique (PI) ci-dessus se replie dans l'espace pour adopter sa conformation tertiaire qui est la forme active du peptide, tout comme la séquence des peptides de l'invention.

Les acides aminés 12, 15, 16, 17, 19, 20, 22, 50, 55, 57, 58, 59, 60 et 65 du peptide de la présente invention sont des acides aminés, ou résidus, impliqués directement ou indirectement dans la liaison aux lipides, c'est à dire qu'il sont impliqués soit dans la structure tridimentionnelle du peptide pour qu'il adopte sa conformation active de reconnaissance, soit dans le site de reconnaissance du lipide.

Les acides aminés J sont les acides aminés, ou résidus, de surface de ce peptide lorsqu'il est dans sa conformation repliée et active. Ces résidus sont disposés dans l'espace de telle sorte qu'ils sont partiellement ou totalement exposés au solvant. Selon la présente invention, ces acides aminés J peuvent être par exemple choisis indépendamment les uns des autres parmi l'ensemble des résidus aminoacides naturels Ala, Arg, Asn, Asp, Cys, Gln, Glu, Gly, His, Ile, Leu, Lys, Met, Orn, Phe, Pro, Ser, Thr Trp, Tyr et Val, et de telle manière que au moins 50% d'entre eux sont des résidus polaires choisis parmi Arg, Asn, Asp, Cys, Gln, Glu, Gly, His, Lys; Orn, Pro, Ser et Thr. Des exemples sont donnés dans la liste de séquences annexée.

Les acides aminés U sont les résidus de coeur de ce peptide. Dans la conformation repliée et active du peptide, ils sont disposés dans l'espace proches les uns des autres et non exposés au solvant. Ils constituent le coeur hydrophobe de la protéine. L'assemblage compact des atomes de ces résidus joue un rôle prédominant pour la stabilité du peptide dans sa conformation active. Ces résidus peuvent être choisis dans la liste d'acides aminés U décrite ci-dessus. Différents exemples de combinaisons de résidus de coeur dans le peptide de séquence (PI) de la présente invention sont donnés dans le tableau (1) ci-dessous :

**Tableau 1**

| | **U⁸** | **U¹¹** | **U¹⁵** | **U²⁵** | **U²⁹** | **B³⁷** | **U⁴⁰** | **U⁴⁴** | **U⁵²** | **U⁵⁶** | **U⁶⁸** | **U⁷²** |
|---|---|---|---|---|---|---|---|---|---|---|---|---|
| **Ex a)** | Val | Leu | Met | Ile | Leu | Arg | Ile | Tyr | Leu | Leu | Val | Leu |
| **Ex b)** | Ala | Ile | Ile | Ile | Leu | Arg | Ile | Tyr | Leu | Leu | Ile | Leu |
| **Ex c)** | Ala | Ile | Ile | Ile | Leu | Arg | Ile | Tyr | Leu | Leu | Met | Val |
| **Ex d)** | Ala | Leu | Met | Leu | Leu | Arg | Ile | Tyr | Leu | Leu | Ile | Met |
| **Ex e)** | Ala | Leu | Met | Ile | Ile | Arg | Val | Tyr | Leu | Leu | Ile | Met |
| **Ex f)** | Ala | Leu | Met | Ile | Ile | Arg | Ile | Phe | Leu | Leu | Ile | Met |
| **Ex g)** | Ala | Leu | Met | Ile | Val | Arg | Ile | Phe | Leu | Leu | Ile | Phe |
| **Ex h)** | Val | Leu | Met | Ile | Leu | Arg | Ile | Phe | Leu | Leu | Ile | Met |
| **Ex i)** | Ala | Leu | Met | Ile | Leu | Arg | Ile | Phe | Leu | Leu | Ile | Met |
| **Ex j)** | Ala | Leu | Met | Ile | Leu | Arg | Ile | Tyr | Leu | Leu | Ala | Ala |
| **Ex k)** | Val | Leu | Met | Ile | Leu | Arg | Ile | Tyr | Leu | Leu | Val | Leu |
| **Ex l)** | Val | Leu | Met | Ile | Leu | Arg | Ile | Phe | Leu | Leu | Val | Leu |

| | | | | | | | | | | | | |
|---|---|---|---|---|---|---|---|---|---|---|---|---|
| (Ex = exemple) | | | | | | | | | | | | |

Le résidu X¹⁸ a pour fonction de maintenir la structure de la boucle Gly-X-Gly dans la forme active du peptide, notamment où les résidus Z⁵⁹ et Z⁶⁵ sont des Glu, de moduler le caractère hydrophobe et lipophile de cette boucle, et d'assurer éventuellement des interactions nouvelles spécifiques avec les phospholipides. Ceci est le cas par exemple des résidus Asn, Cys, Ser, Thr, Trp et Tyr.

Les résidus Z⁵⁹ et Z⁶⁵ peuvent être avantageusement des résidus lysine, ce qui a pour effet de remplacer l'ion calcium par le groupe chargé positivement -NH₃⁺ de la lysine et d'améliorer l'affinité du peptide pour une membrane chargée négativement.

Comme le peptide (PI), le peptide de la présente invention, dans sa forme active comprend trois sites de liaison à un ion calcium où l'ion calcium complexé par ce site constitue un des ligands d'un phospholipide chargé négativement. Le premier de ces sites, appelé site principal, fait intervenir les résidus 15, 18, 19 et 59 en tant que ligands du calcium. Le deuxième de ces sites, appelé site secondaire, fait intervenir les résidus 20 et 22 en tant que ligands du calcium. Le troisième de ces sites, qui est un site secondaire de faible affinité, fait intervenir les résidus 57, 60, et 65 en tant que ligands du calcium.

Les résidus globalement impliqués dans la liaison aux phospholipides sont les résidus 12, 15, 16, 19, 20, 22, 50, 55, 57, 58, 59, 60 et 65. Cette liste inclut des résidus impliqués dans les liaisons du calcium, les phospholipides étant des ligands du calcium.

Ces résidus peuvent bien entendu être remplacés par des résidus remplissant la même fonction en vue du même résultat conformément à la présente invention.

A titre d'exemple, selon l'invention, le peptide de formule (PI) peut être avantageusement une séquence peptidique choisie parmi les séquences peptidique ID n°1 à ID n°10 annexées.

La séquence de la revendication 1 représente les peptides de la présente invention dans leur forme fonctionnelle la plus courte. Il est bien entendu que cette séquence peut comprendre en outre, lié à l'extrémité N-terminale et/ou à l'extrémité C-terminale de la séquence, un ou plusieurs acides aminés, par exemple de 1 à 15 acides aminés, en général de 1 à 10 acides aminés. De toute préférence, ces acides aminés complémentaires ne modifient pas ou peu l'activité des peptides, ou alors l'améliorent.

Par exemple, une petite séquence, appelée ci-dessous séquence de fonctionnalisation peut être utile notamment pour fixer un marqueur sur le peptide, pour fixer une molécule de traitement de maladies sur le peptide et/ou pour fixer ledit peptide sur un support. La longueur de cette séquence de fonctionnalisation sera adaptée suivant son usage. Bien entendu, celle-ci n'interférera de préférence pas avec l'activité du peptide de la présente invention. L'homme du métier saura facilement adapter la longueur et la nature de cette séquence de fonctionnalisation suivant l'utilisation qu'il fera d'un peptide de la présente invention.

Ainsi, selon un premier mode particulier de réalisation de la présente invention, les peptides de la présente invention peuvent comporter, par exemple à leur extrémité N-terminale, une séquence de fonctionnalisation de trois acides aminés. Cette séquence de fonctionnalisation permet une fixation directe du composé de marquage (CI) sur le peptide. Les peptides conformes à ce mode de réalisation peuvent être définis par la séquence (PII) suivante : dans laquelle J, Z, U, X et B sont tels que définis ci-dessus.

Par exemple, J⁻² peut être Gly, J⁻¹ peut être Ser, ou Cys et J⁰ peut être Cys, Thr, Pro, Ser, ou Gln, de préférence J⁰ est Cys . Cette séquence J⁻²J⁻¹-J⁰ peut être choisie par exemple parmi Gly-Ser-Cys-, et Gly-Cys-Ser-. Ainsi, par exemple, chacune des séquences IDn°1 à IDn°10 précitée peut comporter au choix chacune des séquences fonctionnelles précitées. La séquence IDn°12 de la liste de séquences annexée n'est qu'un exemple non limitatif d'une séquence selon la présente invention comportant à son extrémité N-terminale une séquence fonctionnelle de trois acides aminés.

Selon un deuxième mode particulier de réalisation de la présente invention, les peptides peuvent comporter, par exemple à leur extrémité N-terminale, une séquence de fonctionnalisation de quatre acides aminés J⁻³-J⁻²J⁻¹-J⁰ choisie parmi Gly-Ser-Gly-Cys-, Gly-Cys-Gly-Ser, et Gly-Cys-Gly-Cys. Cette séquence de fonctionnalisation est utile par exemple pour une fixation directe du composé de marquage (CI) sur le peptide. Ainsi, par exemple, chacune des séquences IDn°1 à IDn°10 précitées peut comporter au choix chacune des séquences fonctionnelles précitées. Les séquences IDn°11 de la liste de séquences annexée (plusieurs séquences sont regroupées en une seule sous la dénomination IDn°11) ne sont que des exemples non limitatifs de séquence selon la présente invention comportant à son extrémité N-terminale une séquence fonctionnelle de quatre acides aminés.

Selon un troisième mode particulier de réalisation de la présente invention, les peptides peuvent comporter, par exemple à leur extrémité N-terminale, une séquence de fonctionnalisation de sept à onze acides aminés. Cette séquence de fonctionnalisation est également utile pour fixer directement le composé (CI) sur le peptide. Ce mode de réalisation est exposé ci-dessous. Ainsi, par exemple, chacune des séquences IDn°1 à IDn°10 précitées peut comporter au choix chacune des séquences fonctionnelles précitées. On peut aussi remplacer la séquence Gly-Ser-Gly-Cys des séquences IDn°11 à 14 par Gly-Bb1-Gly-Bb2, dans laquelle Bb1 et Bb2 sont indépendamment Cys ou Ser. Les séquences IDn°13 et 14 de la liste de séquences annexée (plusieurs séquences sont regroupées en une seule sous la dénomination IDn°13 ou 14) ne sont que des exemples non limitatifs de tels peptides.

Les peptides de la présente invention ont une affinité suffisante pour le calcium et sont capables de se lier de manière réversible à des effecteurs lipidiques, et notamment à ceux chargés négativement, tel que les phosphatidylsérines, les acides phosphatidiques, les phosphatidyléthanolamines, les phosphatidylglycérols, les cardiolipines et les phosphatidylinositolphosphates.

Il s'agit d'une famille de peptides dont la propriété principale est de reconnaître spécifiquement l'apparition des signaux lipidiques à la surface des membranes cellulaires en relation avec le fonctionnement normal ou pathologique des tissus.

Les peptides de la présente invention peuvent être synthétisés par les procédés classiques de synthèse de la chimie organique ou de la chimie des protéines, ainsi que par recombinaison génétique *in vivo* ou *in vitro,* par génie génétique, etc.

Le peptide selon l'invention peut être synthétisé par synthèse chimique en phase solide dudit peptide. Cette synthèse chimique peut être réalisée par exemple avec un synthétiseur automatique de peptide du type Applied Biosystems, mod.433A. Elle peut être réalisée par exemple en chimie Fmoc qui utilise le groupement fluoremylméthyloxycarbonyle pour la protection temporaire de la fonction α-aminique des acides aminés.

Les éléments techniques pour la réalisation de ce procédé de synthèse peptidique sont connus de l'homme du métier. Ils sont décrits par exemple dans l'ouvrage Solid-Phase Organic Synthesis de Kevin Burgess (Editor) Wiley-Interscience; ISBN: 0471318256; (February 2000).

Le peptide de l'invention peut aussi être fabriqué par recombinaison génétique *in vivo* par exemple au moyen d'un procédé comprenant les étapes suivantes :
a) préparation d'un cDNA comprenant une séquence de base codant pour ledit peptide
b) insertion dudit cDNA dans un vecteur d'expression approprié,
c) transformation d'une cellule hôte appropriée, avec ledit vecteur dans lequel le cDNA a été inséré, pour une réplication du plasmide,
d) fabrication dudit peptide par traduction dudit cDNA dans ladite cellule hôte, et
e) récupération du peptide synthétisé.

Selon l'invention, le vecteur d'expression approprié et la cellule hôte sont choisis selon les techniques habituelles pour la recombinaison génétique. Le vecteur peut être l'un quelconque des plasmides généralement utilisés dans cette technique, par exemple un plasmide tel que le vecteur pGEX-2T. De même la cellule peut être choisie selon les techniques habituelles, il peut s'agir par exemple de *E*. *Coli.*

Lorsqu'une technique de recombinaison génétique *in vitro* est utilisée, les étapes c) et d) du procédé ci-dessus sont remplacées respectivement par les étapes c') d'introduction du vecteur dans lequel le cDNA a été inséré dans un milieu réactionnel adéquat pour une réplication du plasmide, et d') de fabrication dudit peptide par traduction dudit cDNA dans ledit milieu réactionnel adéquat. Le document Jagus, R. and Beckler, G.S. (1998) Overview of eukaryotic in vitro translation and expression systems, Current Protocols in Cell Biology 11.1.1-11.1.13., 1998 by John Wiley & Sons, Inc. décrit des procédées *in vitro* utilisables dans la présente invention.

Selon l'invention, avantageusement dans le composé (CI) de marquage ci-dessus, n=1, et Y est un groupe 3-pyridinyle.

Les composés de formule (CI) peuvent appartenir à diverses familles, une première famille peut être définie comme celle des « éthers d'alkyle », qui répondent à la formule (CII) suivante : 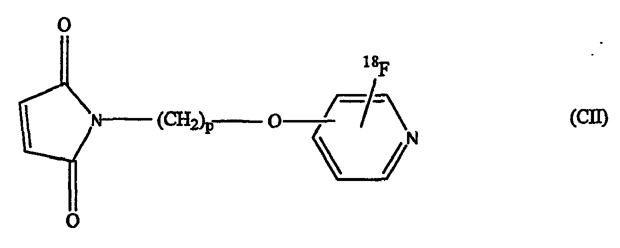 dans laquelle p est un nombre entier de 1 à 10, tel que 2, 3, 4, 5, 6, 7, 8 ou 9.

Les composés préférés de formule (CII) sont choisis parmi les composés suivants :

| | |
|---|---|
| 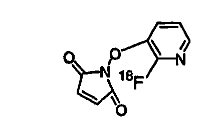 | 1-[(2-[¹⁸F]fluoro-pyridin-3-yloxy)-methyl)-pyrrole-2,5-dione |
| 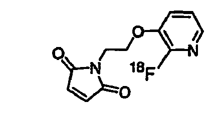 | 1-[2-(2-[¹⁸F] fluoro-pyridin-3-yloxy)-ethyl]-pyrrole-2,5-dione |
| 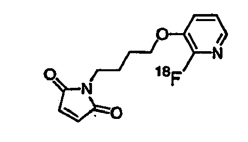 | [1-[4-(2-[¹⁸F]fluoro-pyridin-3-yloxy)-butyl]-pyrrole-2,5-dione |
| 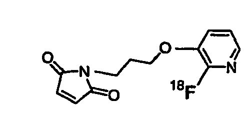 | 1-[3-(2-[¹⁸F]fluoro-pyridin-3-yloxy)-propyl]-pyrrole-2,5-dione |
| 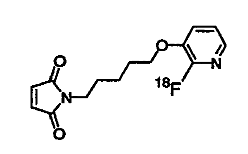 | 1-[5-(2-[¹⁸F]fluoro-pyridin-3-yloxy)-pentyl]-pyrrole-2,5-dione |
| 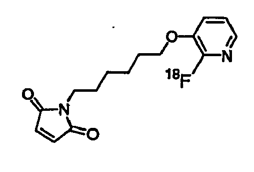 | 1-[6-(2-[¹⁸F]fluoro-pyridin-3-yloxy)-hexyl]-pyrrole-2,5-dione |

Une deuxième famille de composés de formule (CI) peut être définie comme celles des « éthers de phénylalkyle », qui répondent à la formule (CIII) suivante : 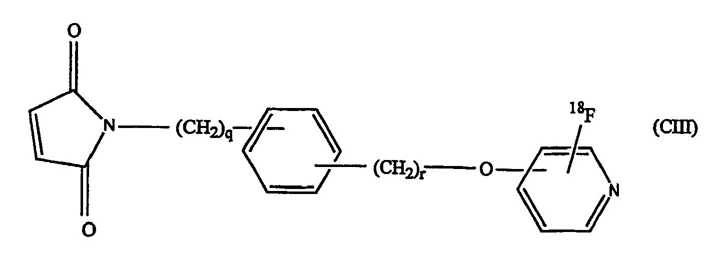 dans laquelle q et r représentent indépendamment un nombre entier de 0 à 10, tels que 0, 1, 2, 3, 4, 5, 6, 7, 8, 9.

Les composés préférés de formule (CIII) sont choisis parmi les composés suivantes :

| | |
|---|---|
| 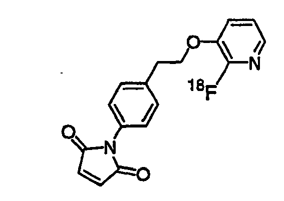 | 1-{4-[2-(2-[¹⁸F]Fluoro-pyridin-3-yloxy)-ethyl]-phenyl}-pyrrole-2,5-dione |
| 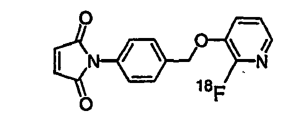 | 1-[4-(2-[¹⁸F]Fluoro-pyridin-3-yloxymethyl)-phenyl]-pyrrole-2,5-dione |
| 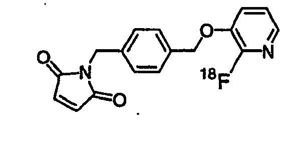 | 1-[4-(2-[¹⁸F]Fluoro-pyridin-3-yloxymethyl)-benzyl]-pyrrole-2,5-dione |

Une troisième famille est celle des composés qui répondent à la formule (CIV) suivante : 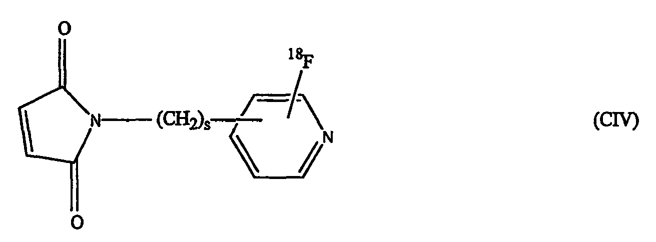 dans laquelle s est un nombre entier de 1 à 10, tel que 2, 3, 4, 5, 6, 7, 8, 9.

Un composé préféré de formule (CIV) est le composé suivant :

| | |
|---|---|
| 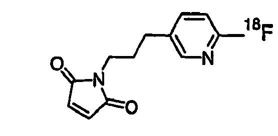 | 1-[3-(6-[¹⁸F]Fluoro-pyridin-3-yl)-propyl]-pyrrole-2,5-dione |

Une quatrième famille est celle des composés qui répondent à la formule (CV) suivante :
dans laquelle t est un nombre entier de 0 à 10, tel que 1, 2, 3, 4, 5, 6, 7, 8, 9 et T est un groupe -CH=CH- ou

Des composés préférés de formule (CV) sont les composés suivantes :

| | |
|---|---|
| 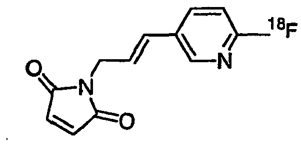 | 1-[3-(6-[¹⁸F]Fluoro-pyridin-3-yl)-allyl]-pyrrole-2,5-dione |
| 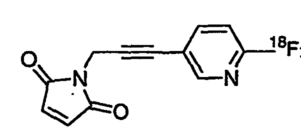 | 1-[3-(6-[¹⁸F]Fluoro-pyridin-3-yl)-prop-2-ynyl]-pyrrole-2,5-dione |

Le composé (CI) de marquage peut être préparé par un procédé dans lequel :
a) on met en contact un composé précurseur de formule (CIa) : 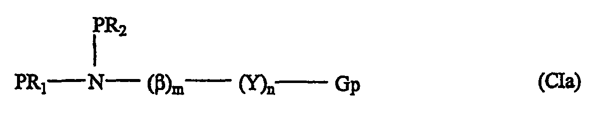 dans laquelle PR₁ et PR₂ représentent indépendamment un atome d'hydrogène ou un groupe protecteur de la fonction amine, à la condition que PR₁ et PR₂ ne soit pas tous deux (simultanément) un atome d'hydrogène, ou bien PR₁ et PR₂ forment ensemble avec l'atome d'azote un groupe protecteur cyclique de la fonction amine, Gp représente un groupe partant susceptible d'être remplacé par un atome de fluor-18, et β, Y, m et n ont la signification déjà donnée plus haut ;avec une source d'ions fluorure F⁻ marqués au [¹⁸F] , pour donner un composé de formule (CIb) _{:} 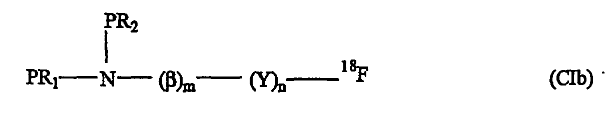
b) on élimine dans le composé (Ib), le ou les groupe(s) protecteur(s) PR₁ et/ou PR₂ de la fonction amine pour donner un composé de formule (Ic) :

   H₂N- (β)ₘ - (Y)ₙ- ¹⁸F (CIc)
c) on fait réagir le composé (CIc) avec un réactif susceptible de donner un groupe maléimido à partir d'un groupe amino, pour obtenir le composé final de formule (CI).

Le procédé selon l'invention est simple, fiable, facile à mettre en oeuvre et peut être aisément robotisé. Il comporte seulement trois étapes dont l'une est une étape extrêmement simple de déprotection.

La durée globale du procédé est faible : à titre d'exemple, elle est généralement de 60 à 120 minutes, de préférence de 75 à 85 minutes.

L'incorporation de l'halogène fluor-18 est réalisée de manière extrêmement efficace avec un fort rendement, par exemple 70 à 100 %, du fait, en particulier, qu'il est effectué sur un groupement hétérocyclique, tel que la pyridine.

Le rendement final de l'ensemble du procédé pour un produit purifié est extrêmement élevé, par exemple de 15 % à 25 % et les quantités potentielles de composé « synthon », en fin de synthèse, sont également très importantes

Dans le composé (CIa), les groupes PR₁ et PR₂ lorsqu'ils sont des groupes protecteurs peuvent être tout groupe protecteur connu en chimie organique. Ils sont choisis, de préférence, parmi les groupes TertioButoxyCarbonyle (BOC) et FluorénylMéthOxyCarbonyle (FMOC).

Lorsque PR₁ et PR₂ forment ensemble avec l'atome d'azote de la fonction amine, un groupe protecteur de celle-ci, ce groupe protecteur peut être, par exemple, un groupe phtalimido.

Dans le composé (CIa), le groupe Gp peut être tout groupe partant susceptible d'être remplacé par un atome de fluor-18 ; Gp est choisi, de préférence, parmi les halogènes, tels que F, Cl, Br, I, les groupes mésyle, tosyle et triflate, lorsque Y est un groupe alkyle ; et Gp est choisi, de préférence, parmi les halogènes, les sels d'ammonium, tel que le triméthylammonium-trifluorométhane sulfonate, et le groupe nitro, lorsque Y est un groupe aromatique ou hétérocyclique.

Dans l'étape a), la source d'ions fluorure marqués au ¹⁸F comprend lesdits ions fluorure et un contre-ion, choisi parmi les cations de grande taille, tels que le rubidium, et le tétrabutylammonium, et les cations de petite taille, tels que le potassium, le sodium et le lithium, lesdits cations de petite taille étant piégés, stabilisés, par exemple, par un cryptand ou un éther couronne, etc..., ledit cryptand ou éther couronne étant adapté au cation de petite taille mis en oeuvre.

Un exemple de cryptand est le produit KRYPTOFIX^{®} K₂₂₂ - (4, 7, 13, 16, 21, 24-hexaoxa-1,10-diazabicyclo[8.8.8]hexacosane) qui piège, par exemple, l'ion potassium.

Le contre-ion ou cation peut être amené sous la forme d'un sel quelconque, par exemple, il peut s'agir de K₂CO₃, dans le cas du potassium.

L'étape a) est généralement réalisée dans un solvant, qui peut être tout solvant adéquat, tel que le DMSO.

L'étape a) peut être réalisée dans des conditions connues de l'homme du métier, avec un chauffage généralement à une température de 50 à 200°C, par exemple, 145°C, pendant une durée généralement de 1 à 30 minutes, par exemple de 4 à 6 minutes.

L'étape b) d'élimination du groupe protecteur de la fonction amine, de déprotection, pour donner le composé de formule (CIc), où le groupe amino est libre, peut être réalisée par tout procédé de déprotection connu. On pourra, par exemple, mettre le composé (CIb) en contact avec du TFA dans le CH₂Cl₂ pendant une durée généralement de 1 à 5, par exemple de 2 minutes.

Il est à noter que le TFA est utilisé généralement, uniquement si le groupe récepteur est enlevé en milieu acide, par exemple lorsque PR₁ = BOC et PR₂ = H.

Dans l'étape c), le réactif susceptible de donner un groupe maléimido à partir d'un groupe amido peut être tout composé connu. Il pourra ainsi être choisi parmi la N-méthoxycarbonylmaléimide et la succinimide.

L'étape c) peut être réalisée dans des conditions connues de l'homme du métier, par exemple dans un solvant, tel que le xylène, le THF, avec un chauffage généralement à une température de 100 à 200°C, par exemple de 190°C, pendant une durée de 1 à 20 minutes, par exemple de 5 minutes.

L'étape c) peut dans un autre mode de réalisation également être réalisée dans un mélange biphasique par exemple de dioxanne et de bicarbonate de sodium aqueux, à température ambiante pendant une durée de 3 à 15 minutes, par exemple 10 minutes ; ce mode de réalisation de l'étape c) offre l'avantage de donner un meilleur rendement et d'être mis en oeuvre à température ambiante, sans qu'il soit nécessaire de chauffer le mélange.

Le composé de formule (CIa) peut répondre à la formule (CIIa) suivante :

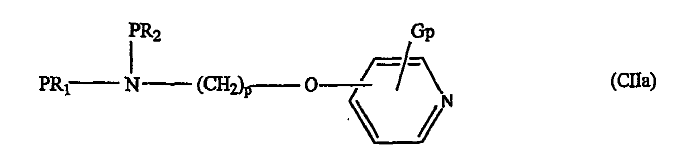

Le composé (CIIa) répond, de préférence, à la formule (CIIb) suivante :

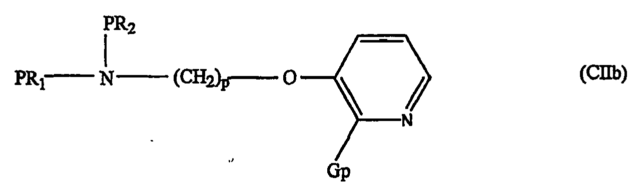

Le composé de formule (CIa) peut, dans un autre mode de réalisation, répondre à la formule (CIIIa) suivante :

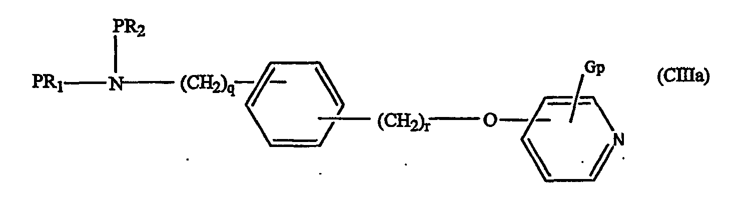

Le composé (CIIIa) répond, de préférence, à la formule (CIIIb) suivante :

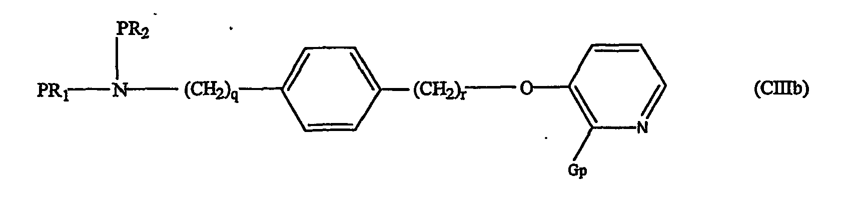

Le composé de formule (CIa) peut, dans encore un autre mode de réalisation, répondre à la formule (CIVa) suivante :

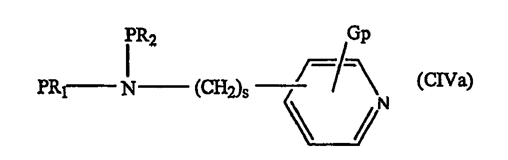

Le composé (CIVa) répond, de préférence, à la formule (CIVb) suivante :

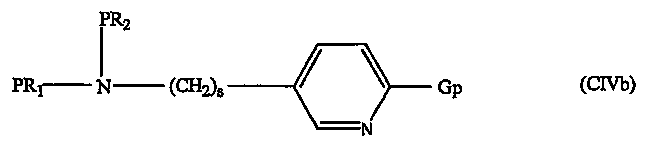

Dans un autre mode de réalisation, le composé de formule (CIa) peut répondre à la formule (CVa) suivante :

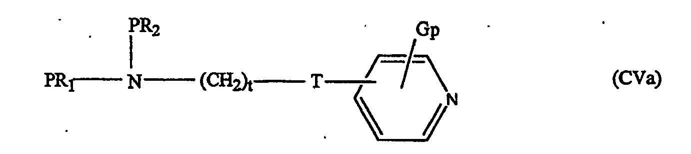

Le composé (CVa) répond, de préférence, à la formule (CVb) suivante :

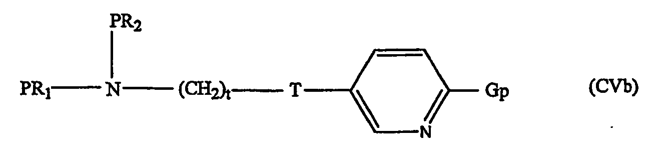

La présente invention se rapporte également à un procédé de synthèse du peptide marqué par le Fluor-18 conforme à la présente invention. Ce procédé de synthèse comprend une étape d'addition d'un composé (CI) défini ci-dessus avec un peptide comprenant la séquence (PI) définie ci-dessus. Il s'agit en effet d'une réaction d'addition réalisée entre la double liaison de la fonction maléimide du composé (CI) et une fonction -SH libre du peptide, notamment la fonction thiol d'une cystéine, du peptide comprenant la séquence peptidique de la revendication 1. L'addition peut être effectuée directement sur une fonction -SH libre de la séquence peptidique de la revendication 1, notamment sur la fonction thiol d'une cystéine de la séquence peptidique, comme décrit ci-dessus. Cette addition peut être faite par exemple dans un solvant acétonitrile/méthanol en proportion respectivement 2:1 en volumes ou dans tout autre solvant approprié pour ce type de réaction d'addition. Il sera bien entendu nécessaire de veiller à ce que le solvant utilise n'affecte pas le peptide de l'invention.

Ce procédé présente donc l'avantage d'être facile à mettre en oeuvre contrairement aux procédés de marquage de l'art antérieur.

Le couplage se fera en préservant l'activité du peptide de la présente invention, et en général aux extrémités ou au niveau des extrémités du peptide de la présente invention, sur des résidus de surface, ou sur une partie de la séquence peptidique différente de la séquence définie à la revendication 1 et notamment sur la séquence (PII) .

La présente invention fournit également un assemblage marqué ayant une affinité pour un phospholipide, comprenant au moins deux peptides comprenant la séquence définie à la revendication 1 identiques ou différents, lesdits peptides étant liés entre eux, et chacun ou l'un seulement de ces peptides étant marqué au moyen d'un composé de marquage (CI) selon l'invention. Ces assemblages peuvent être réalisés par exemple par insertion d'un lien peptidique flexible, par exemple poly-glycine, entre le résidu C-terminal d'un peptide de l'invention et le résidu N-terminal du second peptide et ainsi de suite selon le nombre de peptides mis bout à bout. Ce lien poly-glycine peut être de formule -(Gly)ₙ-, n étant un nombre entier allant de 1 à 12, par exemple supérieur à 4.

Ces assemblages peuvent également être synthétisés par les procédés classiques de synthèse de la chimie organique ou de la chimie des protéines, ainsi que par recombinaison génétique *in vivo* ou *in vitro*, par génie génétique, etc., par exemple par un des procédés précités.

Ces assemblages ont notamment pour but d'augmenter l'affinité des peptides de la présente invention pour le phospholipide, par exemple pour un phospholipide chargé négativement.

L'utilisation d'un peptide marqué ou d'un assemblage marqué de la présente invention peut se faire dans deux directions qui sont la recherche et le diagnostic, et les applications sont très nombreuses.

Les pathologies spécialement visées par la présente invention sont : (i) les troubles de la coagulation sanguine, (ii) les phénomènes d'apoptose consécutifs à l'action de composés chimiques, d'effets physiques comme les radiations ionisantes, d'effets biologiques comme ceux liés à la formation ou la nécrose des tissus cancéreux, outre les phénomènes normaux d'apoptose, (iii) les pathologies inflammatoires, et (iv) les troubles associés aux relations entre les cellules et la matrice extracellulaire et notamment le collagène.

Les peptides de la présente invention présentent en outre un avantage important par rapport aux composés de l'art antérieur : la réversibilité de leurs processus de repliement qui permet leur manipulation à des températures élevées mais compatibles avec la stabilité chimique des peptides, à des fins de modifications chimiques dans le but de développer des molécules utilisables en imagerie.

En outre, en raison de leur faible taille, les peptides de la présente invention peuvent être facilement associés à d'autres protéines soit pour former des protéines chimères multifonctionnelles, soit pour introduire un mécanisme de régulation par des effecteurs autres que les phospholipides de signalisation.

Selon l'invention, les peptides et les assemblages selon l'invention couplés au composé (CI) forment des composés de marquage utilisables par exemple pour un diagnostic *in vivo* ou *in vitro*.

En effet, les peptides de la présente invention peuvent être utilisés pour la détection de pathologies impliquant l'apparition de charges négatives à la surface des cellules et la libération dans le sang de microvésicules : par exemple les troubles de la coagulation, les pathologies inflammatoires aiguës, etc. et l'apoptose.

L'halogène radioactif est le Fluor-18 qui est un radioélément à vie courte car il permet une détection *"in vivo"* de la localisation des zones thrombotiques lors d'accidents vasculaires de toutes sortes, en particulier des foyers apoptotiques et inflammatoires, en utilisant des systèmes d'imagerie appropriés.

Les peptides ou assemblages marqués par le Fluor-18, suivant l'application désirée, peuvent être avantageusement conditionnés sous la forme de trousses de diagnostic. Ainsi, la présente invention fournit également une trousse de diagnostic comprenant un peptide ou un assemblage marqué conforme à la présente invention.

La présente invention fournit également une trousse d'analyse et de détection de charges négatives à la surface de cellules, caractérisée en ce qu'elle comprend un peptide ou un assemblage marqué de la présente invention.

La présente invention fournit également une trousse d'analyse et de détection de microvésicules dans le sang, caractérisée en ce qu'elle comprend un peptide ou un assemblage marqué conforme à la présente invention.

Les peptides marqués par le Fluor-18 selon l'invention peuvent donc être utiles pour la fabrication d'un produit destiné à la détection de centres exposant des lipides chargés négativement à la surface de cellules et/ou la libération dans le sang de microvésicules. Comme précisé ci-dessus, la détection peut être une détection au moyen d'images scintigraphiques acquises en tomographie par émission de positons, du fait que le composé (CI) comprend du ¹⁸F_{.}

Dans leur application, dans le cadre de la « TEP », les composés (CI) et les peptides marqués, selon l'invention, comprenant un atome de fluor-18 montrent de nombreux avantages par rapport aux composés avec un autre halogène radioactif, par exemple l'iode.

En effet, le seul isotope de l'iode émetteur de positons est l'iode-124, qui pourrait permettre la TEP.

Mais, il reste produit à de faibles quantités (qqes mCi contre des curies pour le F-18). Il est aussi difficile à produire. Enfin, l'iode-124 n'est pas un émetteur de positons pur (le fluor-18, 97 %) et décroît par émission beta+ à 25 % seulement et par capture électronique à 75 % ; il possède un grand nombre de raies gamma allant de 0,603 MeV (62 %) à 2,75 MeV (1 %).

L'invention concerne en outre des compositions pour l'analyse et la détection par exemple par tomographie par émission de positons (TEP), ou des compositions pour le diagnostic comprenant un peptide marqué par le fluor 18 tel que décrit plus haut et un véhicule pharmaceutiquement acceptable.

D'autres avantages et caractéristiques de la présente invention apparaîtront encore à la lecture des exemples illustratifs et non limitatifs qui suivent, en référence aux figures en annexe.

### BREVE DESCRIPTION DE LA LISTE DE SEQUENCES

- Les séquences IDn°1 à IDn°14 annexées sont des exemples de peptides comportant la séquence peptidique de la revendication 1 de la présente invention.

En, particulier, les séquences IDn°11, IDn°13 et IDn°14 sont des exemples de peptides comportant la séquence peptidique de la présente invention dans lesquels des mutations ont été introduites pour augmenter l'affinité pour le calcium et les phospholipides.

### BREVE DESCRIPTION DES FIGURES

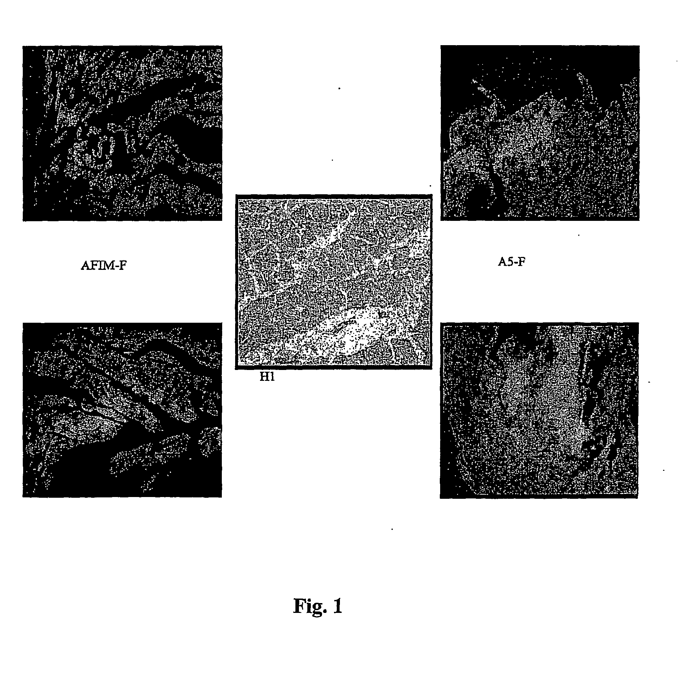

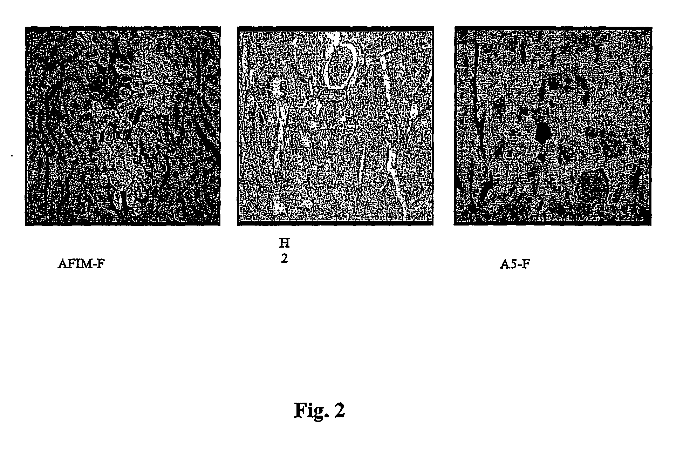

- Les figures 1 et 2 sont des micrographies obtenues à partir de coupes de tissus respectivement d'un coeur apoptotique (figure 1) et d'un rein (figure 2). Ces coupes ont été obtenues d'une part (clichés de gauche) avec des peptides AFIM-fluorescéine (AFIM-F) de la présente invention, d'autre part (clichés de droite) avec de l'annexine 5 -fluorescéine (A5-F) (composé de l'art antérieur) : microscopie de fluorescence, grossissement x 40. Les clichés du centre ont été obtenus avec de l'hématoxyline : microscope en lumière visible, grossissement x 40. Sur la figure 1, les photos du haut et du bas représentent différentes coupes du coeur.

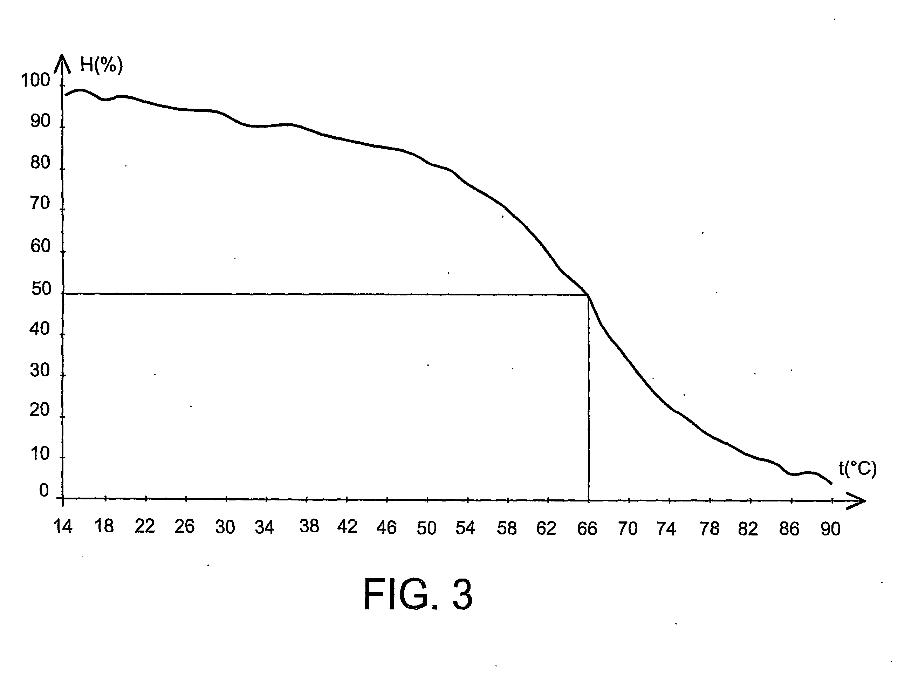

- La figure 3 est un graphique qui représente le taux d'hélicité « H » (en %) d'un peptide selon la présente invention en fonction de la température « t » en °C.

### EXEMPLES

### Exemple 1 : Synthèse par recombinaison génétique: Expression et purification des peptides de séquences ID n°1 à ID n°12 de la présente invention

Les séquences ID n°1 à ID n°14 ont été préparées par surexpression dans *E. Coli* selon le même protocole que celui qui a été décrit par F. Cordier-Ochsenbein et al. dans J. Mol. Biol. 279, 1177-1185.

Les cDNA de chacune de ces séquences ont été préparés en utilisant une réaction de polymérase en chaîne (PCR). Ils ont été insérés dans le vecteur pGEX-2T (Smith & Jonhson, 1998). La figure 2 est un schéma illustrant l'insertion du cDNA dans le vecteur. L'absence de mutations induites par la PCR a été contrôlée par séquençage.

La production des peptides est effectuée en utilisant la souche *E*. *Coli* BL21 contenant le vecteur d'expression décrit plus haut. Après induction par l'isopropylthiogalactopyranoside (IPTG, 100 pm) jusqu'à une densité optique de 1 à 600 nm, la pousse est continuée jusqu'à ce qu'un plateau soit atteint, c'est-à-dire pendant environ 3 heures. Après centrifugation, les bactéries sont re-suspendues dans le tampon de lyse comprenant 50 mM Tris-HCl, pH 8, 10 mM EDTA, 500 mM NaCl, 5% (v/v) glycérol, 1% (v/v) Triton X100, 1 mM dithiothréitol (DTT), 1 mM fluorure de phénylméthylsulfonyl (PMSF) et 20 µg/ml d'aprotinine.

La purification a été effectuée de la façon suivante : après sonication et centrifugation à 10000 g, le surnageant contenant les protéines solubles est incubé avec des billes de glutathion/agarose permettant la liaison spécifique à ces billes de la protéine de fusion GST-domaine. Après lavage avec une solution contenant 1 M NaCl, 50 mM Tris-HCl à pH 8, 70 unités de thrombine par litre de culture sont ajoutés et les séquences sont éluées.

Les séquences sont alors purifiées sur une colonne proRPC (marque de commerce) de type 16/10, fournie par la société Pharmacia en utilisant un système FPLC et un gradient linéaire d'eau de qualité Millipore (marque de commerce) contenant 0,1% (v/v) d'acide trifluoroacétique TFA, et d'acétonitrile contenant 0,1% de TFA. La vitesse d'écoulement est ajustée à 2,5 ml/minute. Les séquences sont ensuite lyophilisées.

Le rendement final pour chaque peptide est d'environ 8 mg de séquence par litre de culture.

### Exemple 2 : Exemple de synthèse chimique de peptides de la présente invention

Les peptides de la présente invention ont été fabriqués dans cet exemple par synthèse chimique en phase solide avec un synthétiseur automatique de peptides Applied Biosystems, mod. 433A, et en chimie Fmoc, qui utilise le groupement Fluorénylméthyloxycarbonyle (Fmoc) pour la protection temporaire de la fonction α-aminique des acides aminés.

Les groupements protecteurs utilisés pour prévenir les réactions secondaires des chaînes latérales des acides aminés, dans cette stratégie Fmoc, ont été le tertio-butyle éther (tBu) pour les résidus Ser, Thr et Tyr ; tertio-butyle ester (OtBu) pour Asp, Glu ; trityle (Trt) pour Gln, Asn, Cys, His ; tertio-butyloxycarbonyle (Boc) pour Lys et 2,2,5,7,8-pentamétylchromane-6-sulfonyle (Pmc) pour Arg.

La réaction de couplage se déroule avec un excès de 10 équivalents d'acides aminés (1 mmol) par rapport à la résine (0,1mmol). L'acide aminé protégé est dissous dans 1 ml de N-méthylpyrollidone (NMP) et 1 ml d'une solution de 1-N-hydroxy-7-azabenzotriazole (HOAt) 1M dans le solvant NMP. 1 ml d'une solution de N,N'-dicyclohexylcarbodiimide (DCC) 1M est alors ajouté. Après 40 à 50 minutes d'activation, l'ester actif formé est transféré dans le réacteur qui contient la résine. Avant cette étape de transfert puis de couplage, la résine est déprotégée de son groupement Fmoc par une solution de 20 % de pipéridine dans le NMP. L'excès de pipéridine est enlevé par lavage à la NMP après 5 à 10 minutes environ.

Pendant la déprotection, la détection des adduits dibenzofulvène-pipéridine à 305 nm permet de suivre le bon déroulement de la synthèse. En effet, la quantification de l'adduit permet d'estimer l'efficacité de la déprotection du groupement Fmoc et par suite du couplage du dernier acide aminé incorporé.

Le clivage de la résine et des groupements protecteurs présents sur les chaînes latérales a été réalisé simultanément par traitement du peptide lié à la résine par de l'acide trifluoroacétique (TFA). Avant d'effectuer le clivage, la résine a été lavée plusieurs fois au dichlorométhane (DCM) et enfin séchée. Le réactif utilisé lors du clivage est un mélange acide contenant 81,5 % de TFA et les piégeurs phénol (5 %), eau (5 %), éthanedithiol (2,5% lorsque le peptide comporte une cystéine) et tri-isopropylsilane (1 %). La résine a été traitée avec ce mélange pendant trois heures sous agitation et à température ambiante, à raison de 100 ml de solution par gramme de résine. Le peptide libre en solution a été récupéré par filtration. Le peptide a été ensuite précipitée et lavée à froid dans l'éther de diisopropyle puis dissous dans de l'acide acétique à 20 % et lyophilisée.

Le peptide récupéré après lyophilisation, le brut de synthèse, se trouve sous forme réduite, c'est à dire que les ponts disulfures intér-chaînes ne sont pas formés.

Le peptide est alors purifié sur une colonne proRPC (marque de commerce) de type 16/10, fournie par la société Pharmacia en utilisant un système FPLC et un gradient linéaire d'eau de qualité Millipore (marque de commerce) contenant 0,1% en volume d'acide trifluoroacétique TFA, et d'acétonitrile contenant 0,1% de TFA. La vitesse d'écoulement est ajustée à 2,5 ml/minute. Le peptide est ensuite lyophilisé.

Les produits obtenus ont été analysés par spectrométrie de masse.

### Exemple 3 : Stabilité des séquences ID n°1 à IDn°14

Cet exemple montre que les peptides de la présente invention constituent des protéines de repliement stables.

### Composition du blanc (témoin):

| | |
|---|---|
| Tris 50 mM, NaCl 150 mM, DTT 1 mM pH8 | 10 µL |
| H₂O | 990 µL |
| Ajusté à pH8 | |

### Composition de l'échantillon :

Echantillon : domaine purifié dans tampon Tris 50 mM,
NaCl 150 mM, pH8 Concentration aprox. : 200 mg.ml.
Domaine : 10 µL soit 300 µM final.
H₂O : 990 µL.
pH mesuré à 7,8.

### Configuration matérielle et logicielle :

Appareil Jobin Yvon CD6.
Logiciel CD-max
Trajet optique de la cuvette de mesure : 1 cm.

La figure 1 annexée représente le taux d'hélicité de AFIM en fonction de la température tel qu'il est mesuré à l'aide du signal de dichroisme circulaire dans l'UV lointain à la longueur d'onde de 220 nm.

Sur cette figure, la valeur du signal à 14°C est prise pour le 100% du contenu en hélice du peptide. La dénaturation thermique du peptide est bien coopérative et démontre qu'à basse température et notamment à 37°C il s'agit d'un peptide convenablement replié et présentant une stabilité améliorée.

### Exemple 4 : Assemblages de deux peptides de la présente invention

Le procédé décrit dans l'exemple 1 ci-dessus est utilisé pour synthétiser une séquence peptidique de séquence IDn°1-(gly)₄₋IDn°1.

Le rendement final pour l'assemblage est d'environ 14 mg/litre de culture.

Cet assemblage peut être marqué par un halogène radioactif selon la présente invention, de la même manière que le peptide seul, par exemple par le procédé décrit ci-dessous.

### Exemple 5 : Synthèse d'un composé de marquage de la présente invention

Dans cet exemple, on décrit la préparation d'un composé de marquage selon l'invention, qui est la 1-[3-(2-[¹⁸F]fluoro-pyridin-3-yloxy)-propyl]-pyrrole-2,5 -dione.

### a) Complexe K [¹⁸F] F-K₂₂₂ -

Afin de récupérer et de recycler la cible d'eau [¹⁸O], on lui fait traverser une résine échangeuse d'anions (AG1x8, de Bio-Rad, 100-200 mesh). L'ion fluorure [¹⁸F] est alors élué de la résine, en utilisant 1,0 mL d'une solution aqueuse de K₂CO₃ à 4,5 mg/mL.

Après addition de 11,0 à 15,0 mg de KRYPTOFIX^{®} K₂₂₂ (4, 7, 13, 16, 21, 24-hexaoxa-1,10-diazobicyclo[8.8.8]hexacosane), la solution résultante est alors doucement concentrée jusqu'à siccité à 145-150°C, sous un courant d'azote pendant 10 minutes pour donner un complexe K [¹⁸F] F-K₂₂₂, pur, sous la forme d'un résidu blanc semi-solide.

### b) 1-[3-(2-[¹⁸F]fluoro-pyridin-3-yloxy)-propyl]-pyrrole-2,5-dione.

Du DMSO, fraîchement distillé (600 µL), contenant 4,0 à 6,0 mg du précurseur de marqueur « nitro » (ester de tertiobutyle de l'acide [3-(2-nitro-pyridin-3-yloxy)-propyl]-carbamique) est ajouté directement dans le tube contenant le complexe K [¹⁸F] -K₂₂₂ séché. Le tube (non scellé) est alors placé dans un bloc de chauffage (à 145°C pendant 4 minutes). Le tube est ensuite refroidi en utilisant un bain glace/eau et la radioactivité restante est mesurée.

85 % à 95 % de l'activité initiale placée dans le récipient est encore présente. Le mélange réactionnel obtenu de couleur sombre, est alors analysé par radiochromatographie. Les rendements d'incorporation sont calculés à partir du radiochromatogramme en CCM et sont définis par le rapport de surface du dérivé ester de tertiobutyle de l'acide [3-(2-[¹⁸F] fluoro-pyridin-3-yloxy)-propyl]-carbamique sur l'activité totale du ¹⁸F fluor-18 (SiO₂-CCM ; éluant : EtOAc ; Rf : : 0,75 et Rf : ion fluorure [¹⁸F] : 0,0). Le mélange réactionnel est dilué avec 1 mL d'eau et transféré sur une cartouche C18 Sep-pak (waters). Le tube est rincé 2 fois avec 1 mL d'eau, qui est également transférée et ajoutée au mélange réactionnel dilué sur la cartouche.

On fait ensuite passer l'ensemble à travers la cartouche. La cartouche est lavée avec 3 mL d'eau et séchée en partie pendant 0,5 minute, en envoyant un courant d'azote.

Le dérivé de l'ester de tertiobutyle de l'acide [3-(2-[¹⁸F]fluoro-pyridin-3-yloxy)-propyl]-carbamique est élué à partir de la cartouche avec 3 mL de dichlorométhane dans une fiole de réaction contenant 0,1 mL de TFA. On utilise 2 fois 1 mL de dichlorométhane pour laver la cartouche et pour transférer complètement le dérivé marqué au [¹⁸F] mentionné ci-dessus (5 % de la quantité de radioactivité totale, impliquée dans le processus de fluoration, reste sur la cartouche). Le rendement d'incorporation est également confirmé après l'élution du Sep-pak par le rapport des valeurs de comptage du CH₂Cl₂ sur radioactivité totale éluée (DMSO/H₂O+CH₂Cl₂) . La solution résultante CH₂Cl₂/TFA (50/1, V/V) est concentrée à siccité (à 65-75°C) sous un courant d'azote modéré pendant 4 à 6 minutes). Le rendement de la déprotection est quantitatif : Nulle molécule, décrite ci-dessus, protégée par BOC ne peut être détecté par radiochromatographie. Le résidu, ci-dessus, est redissous dans 2 mL de CH₂Cl₂ et concentré de nouveau à siccité pour minimiser la présence de TFA (à 65-75°C sous un courant modéré d'azote pendant 4 à 6 minutes). Le résidu est alors dilué avec 0,5 mL de xylène contenant 25 mg de N-méthoxycarbonylmaléimide. Le récipient est alors hermétiquement fermé, chauffé pendant 5 minutes à 190°C (fort reflux), puis refroidi pendant 2 minutes, en utilisant un bain glace/eau. Le mélange réactionnel est alors injecté sur une colonne de HPLC semi-préparative. Elution isocratique [éluant : heptane/EtOAc : 50/50 ; débit : 6,0 mL/minute] qui donne de la 1-(3-(2-[¹⁸F]fluor-pyridin-3-yloxy)-propyl]-pyrrole-2,5-dione marquée, pure, temps de rétention : 7,5 à 8,0 minutes.

Typiquement, 60 à 70 mCi de 1-(3-(2-[¹⁸F]fluor-pyridin-3-yloxy)-propyl]-pyrrole-2,5-dione marquée, pure, peuvent être obtenus en 75 à 85 minutes, à partir de 550-650 mCi d'un lot de production [¹⁸F] F⁻ d'un cyclotron.

### Exemple 5 bis :

Le composé marqué au fluor-18, la 1-[3-(2-[¹⁸F]fluoro-pyridin-3-yloxy)-propyl]-pyrrole-2,5-dione peut également être préparé en répétant les étapes a) et b) du procédé décrit dans l'exemple 5, toujours en utilisant comme précurseur de marquage, le composé « nitro » (ester de tertiobutyle de l'acide [3-(2-nitro-pyridin-3-yloxy)-propyl]-carbamique), mais en modifiant la partie finale de la préparation (étape c)) de la façon suivante (variante selon laquelle l'étape c) est réalisée dans un mélange biphasique de dioxanne et de bicarbonate de sodium aqueux).

Après déprotection de la fonction amine (TFA/CH₂Cl₂), le résidu obtenu après concentration à siccité est repris par 0,250 mL de dioxanne contenant 25 mg de N-méthoxycarbonylmaléimide. A cette solution, 0,750 mL d'une solution aqueuse saturée en bicarbonate de sodium sont ajoutés et la préparation est vortexée à température ambiante pendant 10 minutes. Le mélange réactionnel est ensuite dilué avec 1 mL d'eau et transféré sur une cartouche C18 Sep-pak (Waters). La fiole est rincé 2 fois avec 1 mL d'eau, qui est également transféré et ajouté au mélange réactionnel dilué sur la cartouche. Enfin 8 mL d'eau sont encore ajoutés au mélange réactionnel dilué sur la cartouche. On fait ensuite passer l'ensemble sur la cartouche. La cartouche est lavé avec 3 mL d'eau et séchée en partie pendant 0,5 minutes, en envoyant un courant d'azote. Le dérivé marqué au fluor-18 (1-[3-(2-[¹⁸F]fluoro-pyridin-3-yloxy)-propyl]-pyrrole-2,5-dione) est élué à partir de la cartouche avec 3 mL de dichlorométhane dans une nouvelle fiole vide. On utilise deux fois 1 mL de dichlorométhane pour laver la cartouche et pour transférer complètement le dérivé marqué au [¹⁸F] mentionné ci-dessus. La solution contenant le dérivé marqué au [¹⁸F] mentionné ci-dessus est concentrée (à 65-75°C, sous un courant d'azote modéré pendant 3 à 5 minutes) jusqu'à un volume d'environ 1 mL et injectée sur une colonne de HPLC semi-préparative. La purification est identique à celle décrite dans l'exemple 5.

### Exemple 5 ter :

Le composé marqué au fluor-18, la 1-[3-(2-[¹⁸F]fluoro-pyridin-3-yloxy)-propyl]-pyrrole-2,5-dione peut également être préparé en répétant les étapes a) et b) du procédé décrit dans l'exemple 5 ou 5 bis, mais en utilisant comme précurseur de marquage, le composé « triméthylammonium-trifluoromethane sulfonate » (trifluoromethanesulfonate de [3-(3-*tert-*butoxycarbonylamino-propoxy)-pyridin-2-yl]-trimethyl-ammonium).

### Exemple 6: Marquage d'un peptide de la présente invention par la fluorescéine

Cet exemple, ainsi que l'exemple 7 suivant, ont pour but de démontrer l'efficacité de reconnaissance de sites apoptotiques par les peptides de la présente invention.

Dans les exemples qui suivent, le peptide de la présente invention est appelé AFIM-SH. Il a une séquence peptidique telle que définie par la séquence (PI). Les séquences IDn°1 à IDn°14 sont testées.

La fluorescéine est une molécule qui émet une fluorescence verte d'une longueur d'onde de 525 nm lorsqu'elle est excitée à une longueur d'onde de 488 nm. L'émission de lumière verte est détectée par des caméras ou des photomultiplicateurs. Ce couplage d'AFIM à la fluorescéine permet de détecter la présence des cellules présentant la PS aussi bien *in vitro* que *in vivo* chez des petits animaux.

Selon la présente invention, il est possible de marquer AFIM au niveau des résidus de surface sur toute cystéine qui serait introduite à la place de n'importe quel acide aminé présent à la surface d'AFIM (résidus de surface) pour autant que la fonction de liaison aux membranes lipidiques ne soit pas perturbée. AFIM ainsi modifié est désigné AFIM-SH ci-dessous.

Le couplage de la fluorescéine se fait par l'intermédiaire d'une fonction maléimide représentée ci-dessous sur AFIM par la fonction SH.

La fluorescéine est couplée à une ou plusieurs cystéine(s) de la séquence, de manière covalente, en utilisant une fonction maléimide.

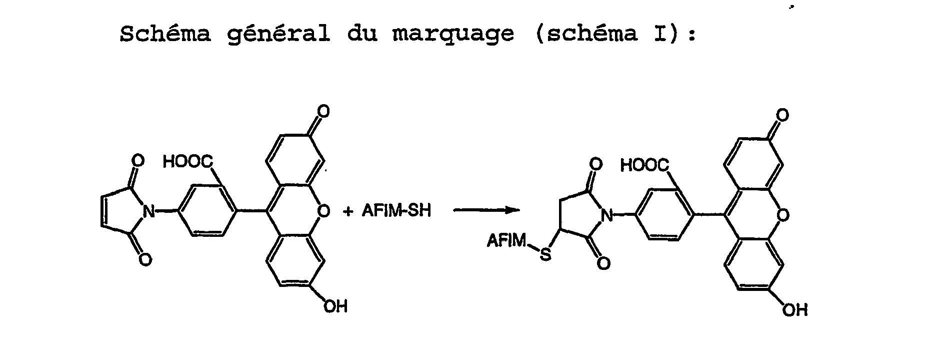

Tout le marquage se fait à une température inférieure à 20°C.

AFIM-SH est en solution dans du tampon Tris (50 mM), NaCl (150 mM), pH = 7,4. 5 équivalents de DTT en solution dans le même tampon sont additionnés à la solution de AFIM-SH. Le milieu est agité durant 30 min.

A l'abri de la lumière: la fluorescéine (5 équivalents d'AFIM-SH + 2 équivalents de DTT) est pesée et dissoute dans du DMF, et additionnée à la solution précédente. Le tout est agité, et la réaction est poursuivie 30 min. Puis le milieu est dilué dans 150 ml de tampon PBS (Phosphate 20 mM, NaCl 150 mM), pH = 7.4, et ultra-filtré sur membrane YM3 (marque de commerce). L'échantillon est re-dilué et ultra-filtré plusieurs fois, en faisant le spectre UV du filtrat.

Lorsqu'il n'y a plus de fluorescéine dans le filtrat (pic à 490 nm), l'échantillon est concentré à quelques ml et conservé au frais à 4°C.

Les produits AFIM-Fluorescéine ont été employés pour détecter des cellules apoptotiques en cytométrie de flux *in vitro,* ainsi que chez des animaux *in vivo* de la manière décrite dans l'exemple 7 suivant.

### Exemple 7 : Résultats de marquages de cellules apoptotiques par les produits AFIM-Fluorescéine de l'exemple 6

Imagerie de cellules cardiaques apoptotiques après un infarctus chez le rat.

Un modèle d'apoptose chez le rat est utilisé comme il est décrit dans l'article paru dans Circulation Res. 1996, 79, 946-956.

Brièvement, quatre rats (300 g chacun) ont été anesthésiés, intubés et ventilés. L'ischémie du myocarde fut provoquée par une occlusion temporaire de l'artère coronaire. Après 30 minutes d'occlusion, l'artère coronaire fut re-perfusée pendant une heure.

A la fin de la période de re-perfusion, les peptides AFIM-Fluorescéine de l'exemple 6 ont été injectés dans la veine jugulaire à raison de 200 µg de peptide pour chacun de deux des rats dans un volume total de 1 ml.

A titre comparatif, 200µg d'annexine 5-Fluorescéine (composés de l'art antérieur) ont été injectés dans les mêmes conditions pour chacun des deux autres rats dans un volume total de 1 ml.

Les rats ont été sacrifiés après 60 minutes.

Cinq organes ont été conservés pour cette étude: le coeur, le poumon, le rein, le foie et le cerveau. Il ont été lavés et rincé en présence de formol. Les organes ont ensuite été déshydratés et imprégnés de paraffine pendant environ 12 heures puis des coupes de 7 µm furent effectuées.

Quelques coupes furent colorées à l'hématoxyline. Les coupes ont été examinées au microscope à fluorescence et les coupes adjacentes colorées à l'hématoxyline furent examinées avec un microscope en lumière visible. Les coupes colorées à l'hématoxyline (marquées H1 et H2 respectivement sur les figures 1 et 2 annexées) permettent une visualisation de l'architecture des tissus et la microscopie de fluorescence de détecter le marquage par AFIM-Fluorescéine (AFIM-F) ou par l'annexine 5-Fluorescéine (A5-F).

La figure 1 annexée montre les images obtenues pour le coeur apoptotique et la figure 2 annexée montre les images obtenues pour le rein.

La figure 1 montre clairement l'excès de fluorescence correspondant à l'accumulation de marqueur au niveau des cellules apoptotiques. Le contraste est visiblement bien meilleur avec AFIM de la présente invention qu'avec l'annexine 5 de l'art antérieur.

La figure 2 montre le marquage du rein lié à l'élimination partielle des produits. Dans le cas de AFIM les glomérules ne semble pas marqués, seule les tubules proximaux sont partiellement marqués. Par contre dans le cas de l'annexine 5 de l'art antérieur l'ensemble du tissus rénal est fortement marqué, ce qui est en accord avec la toxicité rénale observée pour cette protéine.

Les résultats obtenus dans cet exemple démontrent une grande spécificité des peptides de la présente invention pour le marquage des cellules.

Le marquage du peptide AFIM, par exemple de IDn°1 à 10, par la fluorescéine permet donc de détecter efficacement la phosphatidylsérine (PS) présente à la surface externe des cellules impliquées dans des processus physiopathologiques comme la mort cellulaire programmée (apoptose) la coagulation du sang, la réaction inflammatoire.

### Exemple 8 : marquage suivant le procédé de la présente invention de peptides comprenant la séquence (PII) par le composé de marquage (CI)

Dans les exemples qui suivent, le peptide de la présente invention est appelé AFIM-SH. Il a une séquence peptidique telle que définie par la séquence (PII). Les séquences IDn°1 à IDn°14 de la liste de séquences annexées sont testées. Le composé de marquage appelé synthon ¹⁸F fabriqué dans l'exemple 5 (ou 5 bis ou 5 ter) est utilisé dans cet exemple.

AFIM est couplé, spécifiquement au niveau d'une fonction SH de la cystéine J° au synthon ¹⁸F.

Le schéma général du marquage peut être résumé de la manière suivante :

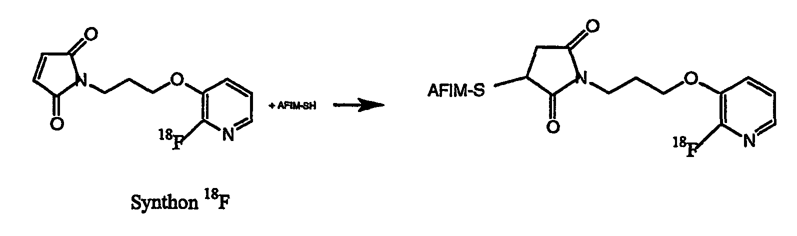

AFIM-SH est en solution dans du tampon Tris (50mM), NaCl (150 mM), pH=7.4. Le synthon ¹⁸F est dissout dans un mélange acétonitrile-méthanol (2/1 v/v), et AFIM-SH est ajouté. Le tout est agité, et la réaction est poursuivie 3 minutes à température ambiante.

Le milieu réactionnel est ensuite transféré sur une colonne de billes maléimide suspendues dans du DMF, et élué avec du tampon PBS.

Le milieu est purifié par HPLC sur colonne de gel d'exclusion, et élue dans du tampon PBS (20mM KH₂PO₄, 150mM NaCl, pH = 7.4).

Le produit, une fois purifié est injecté par voie intraveineuse à des rats.

## Revendications

1. Peptide marqué par le Fluor-18 **caractérisé en ce qu'**il comprend la séquence peptidique suivante : dans laquelle J, Z, U, X et B représentent des acides aminés tels que :
- les acides aminés J sont choisis indépendamment les uns des autres parmi les acides aminés naturels, ou des dérivés de ceux-ci, de telle manière qu'au moins 50% d'entre eux sont des résidus polaires choisis parmi Arg, Asn, Asp, Cys, Gln, Glu, Gly, His, Lys, Orn, Pro, Ser, Thr et Tyr,
- l'acide aminé X¹⁸ est choisi indépendamment des autres acides aminés de la séquence parmi Ala, Asn, Cys, Gln, Gly, His, Ile, Leu, Met, Phe, Ser, Thr, Trp, Tyr et Val,
- les acides aminés Z⁵⁹ et Z⁶⁵ sont choisis indépendamment parmi Glu, Asp, Lys ou Arg,
- les acides aminés U et B de la séquence sont choisis suivant un des exemples a) à j) exposés dans le tableau 1 suivant :
| | **U⁸** | **U¹¹** | **U¹⁵** | **U²⁵** | **U²⁹** | **B³⁷** | **U⁴⁰** | **U⁴⁴** | **U⁵²** | **U⁵⁶** | **U⁶⁸** | **U⁷²** |
|---|---|---|---|---|---|---|---|---|---|---|---|---|
| **Ex a)** | Val | Leu | Met | Ile | Leu | Arg | Ile | Tyr | Leu | Leu | Val | Leu |
| **Ex b)** | Ala | Ile | Ile | Ile | Leu | Arg | Ile | Tyr | Leu | Leu | Ile | Leu |
| **Ex c)** | Ala | Ile | Ile | Ile | Leu | Arg | Ile | Tyr | Leu | Leu | Met | Val |
| **Ex d)** | Ala | Leu | Met | Leu | Leu | Arg | Ile | Tyr | Leu | Leu | Ile | Met |
| **Ex e)** | Ala | Leu | Met | Ile | Ile | Arg | Val | Tyr | Leu | Leu | Ile | Met |
| **Ex f)** | Ala | Leu | Met | Ile | Ile | Arg | Ile | Phe | Leu | Leu | Ile | Met |
| **Ex g)** | Ala | Leu | Met | Ile | Val | Arg | Ile | Phe | Leu | Leu | Ile | Phe |
| **Ex h)** | Val | Leu | Met | Ile | Leu | Arg | Ile | Phe | Leu | Leu | Ile | Met |
| **Ex i)** | Ala | Leu | Met | Ile | Leu | Arg | Ile | Phe | Leu | Leu | Ile | Met |
| **Ex j)** | Ala | Leu | Met | Ile | Leu | Arg | Ile | Tyr | Leu | Leu | Ala | Ala |
| **Ex k)** | Val | Leu | Met | Ile | Leu | Arg | Ile | Tyr | Leu | Leu | Val | Leu |
| **Ex 1)** | Val | Leu | Met | Ile | Leu | Arg | Ile | Phe | Leu | Leu | Val | Leu |
| | | | | | | | | | | | | |
|---|---|---|---|---|---|---|---|---|---|---|---|---|
| (Ex = exemple) | | | | | | | | | | | | |
les exposants des J, Z, U, X et B représentant la position de ces acides aminés dans ladite séquence,
ledit peptide étant marqué directement ou indirectement avec un composé (CI) de formule générale : 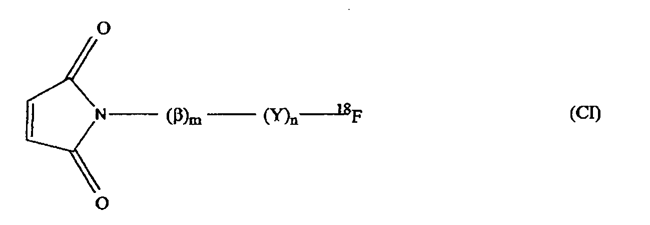 dans laquelle :
- m représente un nombre entier de 0 à 10, tel que 0, 1, 2, 3, 4, 5, 6, 7, 8, 9 ou 10 ;
- n représente un nombre entier de 0 à 10, tel que 0, 1, 2, 3, 4, 5, 6, 7, 8, 9 ou 10 ;
- Y représente un groupe choisi parmi les groupes alkyle, les groupes hétérocycliques monocycliques ou bicycliques choisis parmi les groupes imidazolyle, pyrazolyle, benzimidazolyle, pyridinyle, piridazinyle, pyrimidinyle, pyrazinyle, triazinyle, quinolinyle, isoquinolinyle, cinnolinyle, quinazolinyle, quinoxalinyle, purinyle, Y pouvant être, éventuellement, substitué par un ou plusieurs substituants, chacun de ces substituants étant indépendamment choisi parmi l'hydrogène, les halogènes, les groupes phényle, alkyle en C₁₋₆, alcoxy en C₁₋₆, aryloxy, amino, mono- ou di(alkyle en C₁₋₆) amino, mono- ou di (aryl) amino, thio, alkyle en C₁₋₆-thio, arylthio, formyle, alkyle en C₁₋₆-carbonyle, arylcarbonyle, carbonyle, alcoxy en C₁₋₆-carbonyle, aryloxycarbonyle, alkyle en C₁₋₆-aminocarbonyle, arylaminocarbonyle, trifluorométhyle ;
- β représente un radical de formule :
(γ)ₐ-((CR₁R₂)_{b}-(V)_{c})_{d}-((CR₃R₄)ₑ-(W)_{f})_{g}-
dans laquelle :
- a, b, c, d, e, f, g représentent chacun indépendamment un nombre entier de 0 à 10, tel que 0, 1, 2, 3, 4, 5, 6, 7, 8, 9 ;
- γ, V et W représentent chacun
indépendamment -NR-₁, -O-, -S-, 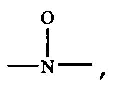 éthynyle, -CR₁=CR₂-, -(C=O)-, -(C=S)-, -C(=NR₁)-, -C(=O)O-, -(C=S)S-, -C(=NR₁)NR₂-, -CR₁R₂-, -CR₁OR₂-, -CR₂NR₂R₃-, où R₁, R₂, R₃ et R₄ sont chacun indépendamment choisis parmi l'hydrogène, les halogènes, les groupes phényle, alkyle en C₁₋₆, alcoxy en C₁₋₆, aryloxy, amino, mono- ou di(alkyle en C₁₋₆)amino, mono- ou di(aryl)amino, thio, alkyle en C₁₋₆-thio, arylthio, formyle, alkyle en C₁₋₆-carbonyle, arylcarbonyle, carbonyle alcoxy en C₁₋₆-carbonyle, aryloxycarbonyle, alkyle en C₁₋₆-aminocarbonyle, arylaminocarbonyle, triflùorométhyle, directement ou indirectement sur une fonction -SH.

2. Peptide marqué par le Fluor-18 selon la revendication 1, dans lequel les acides aminés J sont choisis indépendamment les uns des autres parmi Ala, Arg, Asn, Asp, Cys, Gln, Glu, Gly, His, Ile, Leu, Lys, Met, Phe, Pro, Ser, Thr Trp, Tyr et Val de telle manière que au moins 50% d'entre eux sont des résidus polaires choisis parmi Arg, Asn, Asp, Cys, Gln, Glu, Gly, His, Lys, Pro, Ser et Thr.

3. Peptide marqué par le Fluor-18 comprenant une séquence peptidique choisie parmi la séquence IDn°1, IDn°2, IDn°3, IDn°4, IDn°5, IDn°6, IDn°7, IDn°8, IDn°9, IDn°10, IDn°11, IDn°12, IDn°13 et IDn°14 de la liste de séquences annexée et dans lequel ledit peptide est marqué directement ou indirectement avec un composé (CI) de formule générale : 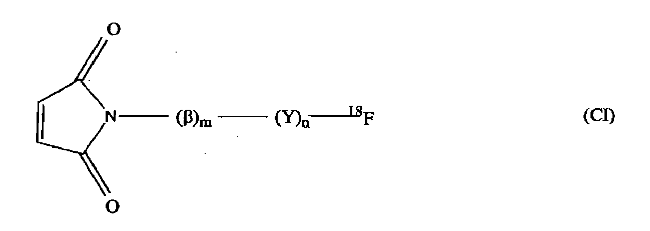 dans laquelle :
- m représente un nombre entier de 0 à 10, tel que 0, 1, 2, 3, 4, 5, 6, 7, 8, 9 ou 10 ;
- n représente un nombre entier de 0 à 10, tel que 0, 1, 2, 3, 4, 5, 6, 7, 8, 9 ou 10 ;
- Y représente un groupe choisi parmi les groupes alkyle, les groupes hétérocycliques monocycliques ou bicycliques choisis parmi les groupes imidazolyle, pyrazolyle, benzimidazolyle, pyridinyle, piridazinyle, pyrimidinyle, pyrazinyle, triazinyle, quinolinyle, isoquinolinyle, cinnolinyle, quinazolinyle, quinoxalinyle, purinyle, Y pouvant être, éventuellement, substitué par un ou plusieurs substituants, chacun de ces substituants étant indépendamment choisi parmi l'hydrogène, les halogènes, les groupes phényle, alkyle en C₁₋₆, alcoxy en C₁₋₆, aryloxy, amino, mono- ou di(alkyle en C₁₋₆) amino, mono- ou di(aryl)amino, thio, alkyle en C₁₋₆-thio, arylthio, formyle, alkyle en C₁₋₆-carbonyle, arylcarbonyle, carbonyle, alcoxy en C₁₋₆-carbonyle, aryloxycarbonyle, alkyle en C₁₋₆-aminocarbonyle, arylaminocarbonyle, trifluorométhyle ;
- β représente un radical de formule :
(γ)ₐ-((CR₁R₂)_{b}-(V)_{c})_{d}-((CR₃R₄)ₑ-(W)_{f})_{g}-
dans laquelle :
- a, b, c, d, e, f, g représentent chacun indépendamment un nombre entier de 0 à 10, tel que 0, 1, 2, 3, 4, 5, 6, 7, 8, 9 ;
- γ, V et W représentent chacun indépendamment -NR-₁, -O-, -S-, 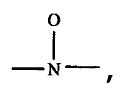 éthynyle, -CR₁=CR₂-, - (C=O)-, -(C=S)-, -C(=NR₁)-, -C(=O)O-, -(C=S)S-, -C(=NR₁)NR₂-, -CR₁R₂-, -CR₁OR₂-, -CR₁NR₂R₃-, où R₁, R₂, R₃ et R₄ sont chacun indépendamment choisis parmi l'hydrogène, les halogènes, les groupes phényle, alkyle en C₁₋₆, alcoxy en C₁₋₆, aryloxy, amino, mono- ou di(alkyle en C₁₋₆)amino, mono- ou di (aryl)amino, thio, alkyle en C₁₋₆-thio, arylthio, formyle, alkyle en C₁₋₆-carbonyle, arylcarbonyle, carbonyle alcoxy en C₁₋₆-carbonyle, aryloxycarbonyle, alkyle en C₁₋₆-aminocarbonyle, arylaminocarbonyle, trifluorométhyle, directement ou indirectement sur une fonction -SH.

4. Peptide marqué au Fluor-18 selon l'une quelconque des revendications 1 à 3, comprenant en outre, liée à son extrémité N-terminal, la séquence d'acides aminés choisie parmi Gly-Ser-Cys et Gly-Cys-Ser.

5. Peptide marqué au Fluor-18 selon l'une quelconque des revendications 1 à 3, comprenant en outre, liée à son extrémité N-terminale, une séquence d'acides aminés choisie parmi Gly-Ser-Gly-Cys, Gly-Cys-Gly-Ser, et Gly-Cys-Gly-Cys.

6. Peptide marqué par le Fluor-18 selon l'une quelconque des revendications 1 à 5, dans lequel le peptide est marqué directement avec le composé (CI) par couplage de la fonction maléimide du composé (CI) avec une fonction -SH libre dudit peptide, par exemple la fonction thiol d'une cystéine du peptide.

7. Peptide marqué par le Fluor-18 selon l'une quelconque des revendications 1 à 5, dans lequel le peptide est marqué directement avec le composé (CI) par couplage de la fonction maléimide du composé (CI) avec une fonction -SH libre de la séquence peptidique (PI), par exemple la fonction thiol d'une cystéine de la séquence peptidique.

8. Peptide marqué par le Fluor-18 selon l'une quelconque des revendications 1 à 5, dans lequel, dans le composé de formule (CI), n=1, et Y est un groupe 3-pyridinyle.

9. Peptide marqué par le Fluor-18 selon la revendication 8, dans lequel le composé (CI) répond à la formule (CII) suivante : 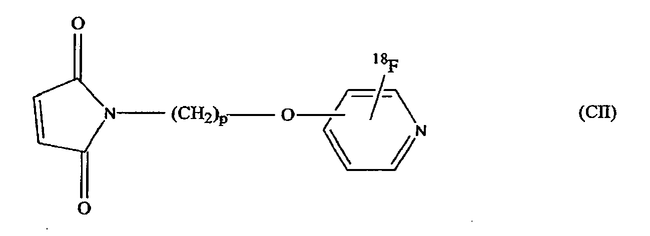 dans laquelle p est un nombre entier de 1 à 10, tel que 2, 3, 4, 5, 6, 7, 8 ou 9.

10. Peptide marqué par le Fluor-18 selon la revendication 9 dans lequel le composé de formule (CII) est choisi parmi :
- la 1-[2-(2-[¹⁸F]fluoro-pyridin-3-yloxy)-éthyl]-pyrrole-2,5-dione ;
- la 1-[4-(2-[¹⁸F]fluoro-pyridin-3-yloxy)-butyl]-pyrrole-2,5-dione ;
- la 1-[5-(2-[¹⁸F]fluoro-pyridin-3-yloxy)-pentyl]-pyrrole-2,5-dione ;
- la 1-[6-(2-[¹⁸F]fluoro-pyridin-3-yloxy)-hexyl]-pyrrole-2,5-dione ;
- la 1-[(2-[¹⁸F]fluoro-pyridin-3-yloxy)-méthyl]-pyrrole-2,5-dione ;
- la 1-[3-(2-[¹⁸F]fluoro-pyridin-3-yloxy)-propyl]-pyrrole-2,5-dione.

11. Peptide marqué par le Fluor-18 selon la revendication 8 dans lequel le composé de formule (CI) répond à la formule (CIII) suivante : 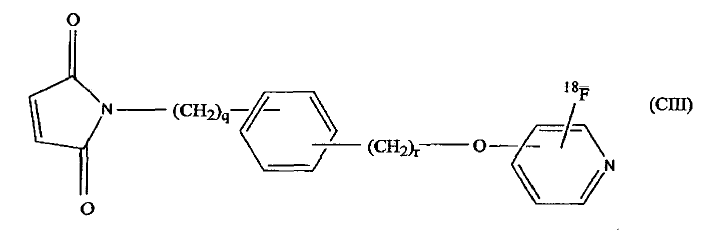 dans laquelle q et r représentent indépendamment un nombre entier de 0 à 10, tels que 0, 1, 2, 3, 4, 5, 6, 7, 8, 9.

12. Peptide marqué par le Fluor-18 selon la revendication 11 dans lequel le composé de formule (CIII) est choisi parmi :
- la 1-{4-[2-(2-[¹⁸F]fluoro-pyridin-3-yloxy)-ethyl]-phenyl}-pyrrole-2,5-dione ;
- la 1-[4-(2--[¹⁸F]fluoro-pyridin-3-yloxymethyl)-phenyl]-pyrrole-2,5-dione ;
- la 1-[4-(2--[¹⁸F]fluoro-pyrridin-3-yloxymethyl) -benzyl]-pyrrole-2,5-dione.

13. Peptide marqué par le Fluor-18 selon la revendication 8 dans lequel le composé de formule (CI) répond à la formule (CIV) suivante :

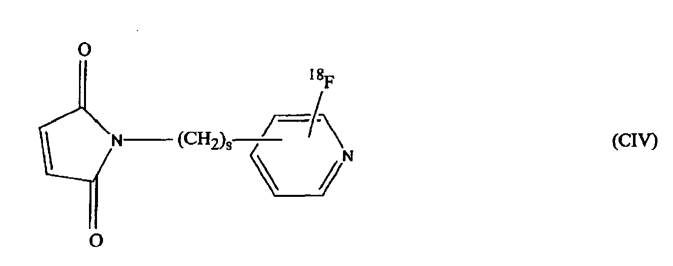

14. Peptide marqué par le Fluor-18 selon la revendication 13, dans lequel le composé de formule (CIV) est la 1-[3-(6-[¹⁸F]fluoro-pyridin-3-yl)-propyl]-pyrrole-2,5-dione.

15. Peptide marqué par le Fluor-18 selon la revendication 8 dans lequel le composé de formule (CI) répond à la formule (CV) suivante : 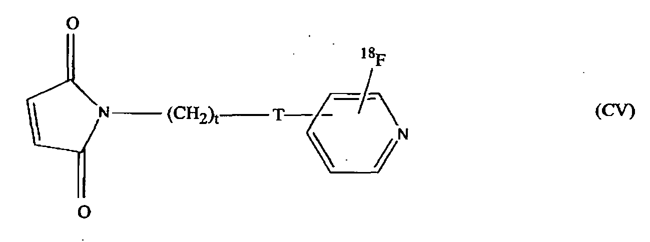 dans laquelle t est un nombre entier de O à 10, tel que 1, 2, 3, 4, 5, 6, 7, 8, 9 et T est un groupe -CH=CH- ou -C≡C-.

16. Peptide marqué par le Fluor-18 selon la revendication 15, dans lequel le composé (CV) est choisi parmi :
- la 1-[3-(6-[¹⁸Flfluoro-pyridin-3-yl)-allyl]-pyrrole-2,5-dione ;
- la 1-[3-(6-[¹⁸F]fluoro-pyridin-3-yl)-prop-2-ynyl]-pyrrole-2,5-dione.

17. Peptide marqué par le Fluor-18 selon l'une quelconque des revendiations 3 à 5,
dans lequel la séquence peptidique est choisie parmi la séquence IDn°1, IDn°2, IDn°3, IDn°4, IDn°5, IDn°6, IDn°7, IDn°8, IDn°9, IDn°10, IDn°11, IDn°12, IDn°13 et IDn°14 de la liste de séquences annexée,
dans lequel le composé (CI) est choi-si parmi :
- la 1-[3-(6-[¹⁸F]fluoro-pyridin-3-yl)-allyl]-pyrrole-2,5-dione ;
- la 1-[3-(6-[¹⁸F]fluoro-pyridin-3-yl)-prop-2-ynyl]-pyrrole-2,5-dione.

18. Procédé de synthèse d'un peptide marqué par un halogène radioactif selon l'une quelconque des revendications 1 à 5, comprenant une étape d'addition d'un composé (CI) de formule générale : 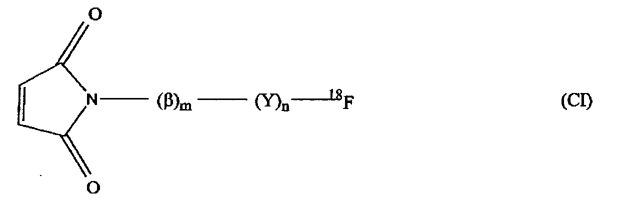 dans laquelle :
- m représente un nombre entier de 0 à 10, tel que 0, 1, 2, 3, 4, 5, 6, 7, 8, 9 ou 10 ;
- n représente un nombre entier de 0 à 10, tel que 0, 1, 2, 3, 4, 5, 6, 7, 8, 9 ou 10 ;
- Y représente un groupe choisi parmi les groupes alkyle, les groupes hétérocycliques monocycliques ou bicycliques choisis parmi les groupes imidazolyle, pyrazolyle, benzimidazolyle, pyridinyle, piridazinyle, pyrimidinyle, pyrazinyle, triazinyle, quinolinyle, isoquinolinyle, cinnolinyle, quinazolinyle, quinoxalinyle, purinyle, Y pouvant être, éventuellement, substitué par un ou plusieurs substituants, chacun de ces substituants étant indépendamment choisi parmi l'hydrogène, les halogènes, les groupes phényle, alkyle en C₁₋₆, alcoxy en C₁₋₆, aryloxy, amino, mono- ou di(alkyle en C₁₋₆)amino, mono- ou di(aryl)amino, thio, alkyle en C₁₋₆-thio, arylthio, formyle, alkyle en C₁₋₆-carbonyle, arylcarbonyle, carbonyle, alcoxy en C₁₋₆-carbonyle, aryloxycarbonyle, alkyle en C₁₋₆-aminocarbonyle, arylaminocarbonyle, trifluorométhyle ;
- β représente un radical de formule :
(γ)ₐ-((CR₁R₂)_{b}-(V)_{c})_{d}- ((CR₃R₄)ₑ-(W)_{f})_{g}-
dans laquelle :
- a, b, c, d, e, f, g représentent chacun indépendamment un nombre entier de 0 à 10, tel que 0, 1, 2, 3, 4, 5, 6, 7, 8, 9 ;
- γ, V et W représentent chacun indépendamment -NR-₁, -O-, -S-, 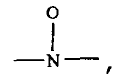 éthynyle, -CR₁=CR₂-, -(C=O)-, -(C=S)-, -C (=NR₁) -, -C(=O)O-, - (C=S) S-, -C(=NR₁) NR₂-, -CR₁R₂-, -CR₁OR₂-, -CR₁NR₂R₃-, où R₁, R₂, R₃ et R₄ sont chacun indépendamment choisis parmi l'hydrogène, les halogènes, les groupes phényle, alkyle en C₁₋₆, alcoxy en C₁₋₆, aryloxy, amino, mono- ou di(alkyle en C₁₋₆)amino, mono- ou di(aryl)amino, thio, alkyle en C₁₋₆-thio, arylthio, formyle, alkyle en C₁₋₆-carbonyle, arylcarbonyle, carbonyle alcoxy en C₁₋₆-carbonyle, aryloxycarbonyle, alkyle en C₁₋₆-aminocarbonyle, arylaminocarbonyle, trifluorométhyle ;
directement ou indirectement sur une fonction -SH d'un peptide.

19. Procédé selon la revendication 18, dans lequel l'addition est effectuée directement sur une fonction -SH libre de la séquence peptidique (PI) ; par exemple la fonction thiol d'une cystéine de la séquence peptidique.

20. Trousse d'analyse et de détection de charges négatives à la surface de cellules, **caractérisée en ce qu'**elle comprend un peptide marqué par le Fluor-18 selon l'une quelconque des revendications 1 à 17.

21. Trousse de diagnostic comprenant un peptide marqué par le Fluor-18 selon l'une quelconque des revendications 1 à 17.

22. Trousse d'analyse et de détection de microvésicules dans le sang, **caractérisée en ce qu'**elle comprend un peptide marqué par le Fluor-18 selon l'une quelconque des revendications 1 à 17.

23. Utilisation d'un peptide marqué par le Fluor-18 selon l'une quelconque des revendications 1 à 17 pour la fabrication d'un produit destiné à la détection de centres exposant des lipides chargés négativement à la surface de cellules et/ou la libération dans le sang de microvésicules.

24. Utilisation selon la revendication 23, dans laquelle la détection est une détection au moyen d'images scintigraphiques acquises en tomographie par émission de positons (TEP).

25. Composition pour l'analyse et la détection par exemple par tomographie par émission de positons (TEP) ayant un peptide marqué par le Fluor-18 selon l'une quelconque des revendications 1 à 17 et un véhicule pharmaceutiquement acceptable.

26. Composition pour le diagnostic comprenant un peptide marqué par le Fluor-18 selon l'une quelconque des revendications 1 à 17 et un véhicule pharmaceutiquement acceptable.

## Claims

1. Peptide labelled with fluorine-18, **characterized in that** it comprises the following peptide sequence : in which J, Z, U, X and B represent amino acids such that:
- the amino acids J are chosen independently of each other from natural amino acids, or derivatives thereof, in such a manner that at least 50% of them are polar residues chosen from Arg, Asn, Asp, Cys, GIn, Glu, Gly, His, Lys, Orn, Pro, Ser, Thr and Tyr,
- the amino acid X¹⁸ is chosen independently of the other amino acids of the sequence from Ala, Asn, Cys, GIn, Gly, His, Ile, Leu, Met, Phe, Ser, Thr, Trp, Tyr and Val,
- the amino acids 2⁵⁹ and 2⁶⁵ are chosen independently from Glu, Asp, Lys and Arg,
- the amino acids U and B of the sequence are chosen according to one of the examples a) to j) disclosed in table 1 below:
| | U⁸ | U¹¹ | U¹⁵ | U²⁵ | U²⁹ | B³⁷ | U⁴⁰ | U⁴⁴ | U⁵² | U⁵⁶ | U⁶⁸ | U⁷² |
|---|---|---|---|---|---|---|---|---|---|---|---|---|
| Ex a) | Val | Leu | Met | Ile | Leu | Arg | Ile | Tyr | Leu | Leu | Val | Leu |
| Ex b) | Ala | Ile | Ile | Ile | Leu | Arg | Ile | Tyr | Leu | Leu | Ile | Leu |
| Ex c) | Ala | Ile | Ile | Ile | Leu | Arg | Ile | Tyr | Leu | Leu | Met | Val |
| Ex d) | Ala | Leu | Met | Leu | Leu | Arg | Ile | Tyr | Leu | Leu | Ile | Met |
| Ex e) | Ala | Leu | Met | Ile | Ile | Arg | Val | Tyr | Leu | Leu | Ile | Met |
| Ex f) | Ala | Leu | Met | Ile | Ile | Arg | Ile | Phe | Leu | Leu | Ile | Met |
| Ex g) | Ala | Leu | Met | Ile | Val | Arg | Ile | Phe | Leu | Leu | Ile | Phe |
| Ex h) | Val | Leu | Met | Ile | Leu | Arg | Ile | Phe | Leu | Leu | Ile | Met |
| Ex i) | Ala | Leu | Met | Ile | Leu | Arg | Ile | Phe | Leu | Leu | Ile | Met |
| Ex j) | Ala | Leu | Met | Ile | Leu | Arg | Ile | Tyr | Leu | Leu | Ala | Ala |
| Ex k) | Val | Leu | Met | Ile | Leu | Arg | Ile | Tyr | Leu | Leu | Val | Leu |
| Ex 1) | Val | Leu | Met | Ile | Leu | Arg | Ile | Phe | Leu | Leu | Val | Leu |
| | | | | | | | | | | | | |
|---|---|---|---|---|---|---|---|---|---|---|---|---|
| (Ex = example) | | | | | | | | | | | | |
the superscripts of J, Z, U, X and B representing the positions of these amino acids in said sequence, said peptide being labelled directly or indirectly with a compound (CI) of general formula: 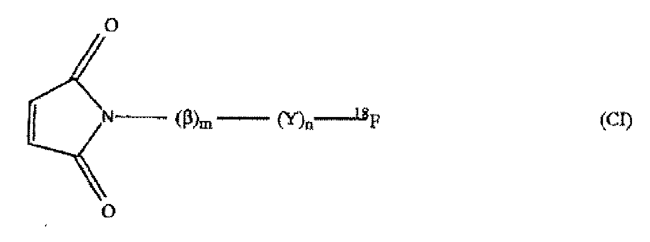 in which:
- m represents an integer from 0 to 10, such as 0, 1, 2, 3, 4, 5, 6, 7, 8, 9 or 10;
- n represents an integer from 0 to 10, such as 0, 1, 2, 3, 4, 5, 6, 7, 8, 9 or 10;
- Y represents a group chosen from alkyl groups, monocyclic or bicyclic heterocyclic groups chosen from imidazolyl, pyrazolyl, benzimidazolyl, pyridinyl, piridazinyl, pyrimidinyl, pyrazinyl, triazinyl, quinolinyl, isoquinolinyl, cinnolinyl, quinazolinyl, quinoxalinyl and purinyl groups, it being possible for Y to be optionally substituted with one or more substituents, each of these substituents being chosen independently from hydrogen, (nonradioactive) halogens, phenyl, C₁₋₆ alkyl, C₁₋₆ alkoxy, aryloxy, amino, mono- or di (C₁₋₆ alkyl) amino, mono- or di(aryl)amino, thio, C₁₋₆ alkylthio, arylthio, formyl, C₁₋₆ alkylcarbonyl, arylcarbonyl, carbonyl, C₁₋₆ alkoxycarbonyl, aryloxycarbonyl, C₁₋₆ alkylaminocarbonyl, arylaminocarbonyl and trifluoromethyl groups;
- β represents a radical of formula:
(γ)ₐ- ((CR₁R₂)_{b}- (V) c)_{d}- ((CR₃R₄)ₑ - (W)_{f})_{g}-
in which:
- a, b, c, d, e, f, g each independently represent an integer from 0 to 10, such as 0, 1, 2, 3, 4, 5, 6, 7, 8, 9;
- γ, V and W each independently represent -NR-₁, -O-, -S-, 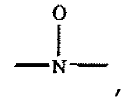 ethynyl, -CR₁=CR₂, -(C=O)-, -(C=S)-, -C (=NR₁) -, -C(=O)O-, - (C=S) S-, -C(=NR₁)NR₂-, -CR₁R₂-, -CR₁OR₂-, -CR₁NR₂R₃-, where R₁, R₂, R₃ and R₄ are independently chosen from hydrogen, halogens, phenyl, C₁₋₆ alkyl, C₁₋₆ alkoxy, aryloxy, amino, mono- or di (C₁₋₆ alkyl) amino, mono- or di(aryl)amino, thio, C₁₋₆ alkylthio, arylthio, formyl, C₁₋₆ alkylcarbonyl, arylcarbonyl, carbonyl (C₁₋₆)alkoxycarbonyl, aryloxycarbonyl, C₁₋₆ alkylaminocarbonyl, arylaminocarbonyl and trifluoromethyl groups, directly or indirectly on an -SH functional group.

2. Peptide labelled with fluorine-18 according to Claim 1, in which the amino acids J are chosen independently of each other from Ala, Arg, Asn, Asp, Cys, Gln, Glu, Gly, His, Ile, Leu, Lys, Met, Phe, Pro, Ser, Thr, Trp, Tyr, and Val in such a manner that at least 50% of them are polar residues chosen from Arg, Asn, Asp, Cys, Gln, Glu, Gly, His, Lys, Pro, Ser and Thr.

3. Peptide labelled with fluorine-18 comprising a peptide sequence chosen from the sequence ID No. 1, ID No. 2, ID No. 3, ID No. 4, ID No. 5, ID No. 6, ID No. 7, ID No. 8, ID No. 9, ID No. 10, ID No. 11, ID No. 12, ID No. 13 and ID No. 14 of the appended sequence listing and in which said peptide is labelled directly or indirectly with a compound (CI) of general formula: 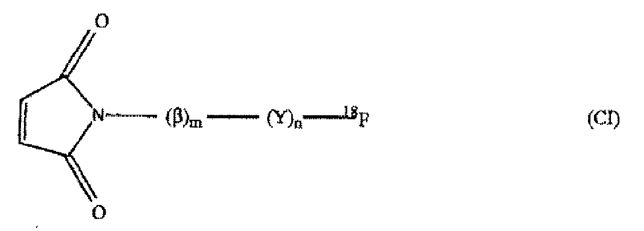 in which:
- m represents an integer from 0 to 10, such as 0, 1, 2, 3, 4, 5, 6, 7, 8, 9 or 10;
- n represents an integer from 0 to 10, such as 0, 1, 2, 3, 4, 5, 6, 7, 8, 9 or 10;
- Y represents a group chosen from alkyl groups, monocyclic or bicyclic heterocyclic groups chosen from imidazolyl, pyrazolyl, benzimidazolyl, pyridinyl, piridazinyl, pyrimidinyl, pyrazinyl, triazinyl, quinolinyl, isoquinolinyl, cinnolinyl, quinazolinyl, quinoxalinyl and purinyl groups, it being possible for Y to be optionally substituted with one or more substituents, each of these substituents being chosen independently from hydrogen, (nonradioactive) halogens, phenyl, C₁₋₆ alkyl, C₁₋₆ alkoxy, aryloxy, amino, mono- or di (C₁₋₆ alkyl) amino, mono- or di(aryl)amino, thio, C₁₋₆ alkylthio, arylthio, formyl, C₁₋₆ alkylcarbonyl, arylcarbonyl, carbonyl, C₁₋₆ alkoxycarbonyl, aryloxycarbonyl, C₁₋₆ alkylaminocarbonyl, arylaminocarbonyl and trifluoromethyl groups;
- β represents a radical of formula:
(γ)ₐ- ((CR₁R₂)_{b}-(V) c)_{d}-((CR₃R₄)ₑ-(W)_{f})_{g}-
in which:
- a, b, c, d, e, f, g each independently represent an integer from 0 to 10, such as 0, 1, 2, 3, 4, 5, 6, 7, 8, 9;
- γ, V and W each independently represent -NR-₁, -O-, -S-, 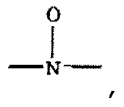 ethynyl, -CR₁=CR₂, -(C=O)-, -(C=S)-, -C (=NR₁) -, -C(=O)O-, - (C=S) S-, -C (=NR₁) NR₂-, -CR₁R₂-, -CR₁OR₂-, -CR₁NR₂R₃-, where R₁, R₂, R₃ and R₄ are independently chosen from hydrogen, halogens, phenyl, C₁₋₆ alkyl, C₁₋₆ alkoxy, aryloxy, amino, mono- or di (C₁₋₆ alkyl) amino, mono- or di(aryl)amino, thio, C₁₋₆ alkylthio, arylthio, formyl, C₁₋₆ alkylcarbonyl, arylcarbonyl, carbonyl (C₁₋₆) alkoxycarbonyl, aryloxycarbonyl, C₁₋₆ alkylaminocarbonyl, arylaminocarbonyl and trifluoromethyl groups, directly or indirectly on an -SH functional group.

4. Peptide labelled with fluorine-18 according to any one of Claims 1 to 3, additionally comprising, linked to its N-terminal end, the amino acid sequence chosen from Gly-Ser-Cys and Gly-Cys-Ser.

5. Peptide labelled with fluorine-18 according to any one of Claims 1 to 3, additionally comprising, linked to its N-terminal end, an amino acid sequence chosen from Gly-Ser-Gly-Cys, Gly-Cys-Gly-Ser and Gly-Cys-Gly-Cys.

6. Peptide labelled with fluorine-18 according to any one of Claims 1 to 5, in which the peptide is labelled directly with the compound (CI) by coupling the maleimide functional group of the compound (CI) with a free -SH functional group of the said peptide, for example the thiol functional group of a cystein of the peptide.

7. Peptide labelled with fluorine-18 according to any one of Claims 1 to 5, in which the peptide is labelled directly with the compound (CI) by coupling the maleimide functional group of the compound (CI) with a free -SH functional group of the peptide sequence (PI), for example the thiol functional group of a cystein of the peptide sequence.

8. Peptide labelled with fluorine-18 according to any one of Claims 1 to 5, in which, in the compound of formula (CI), n = 1, and Y is a 3-pyridinyl group.

9. Peptide labelled with fluorine-18 according to Claim 8, in which the compound (CI) corresponds to the following formula (CII): 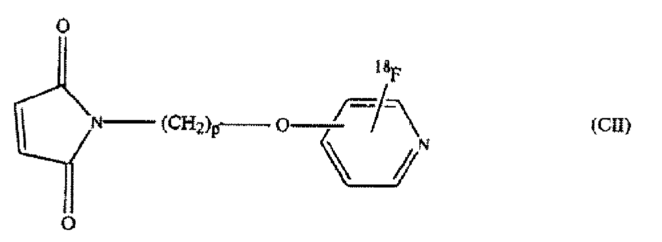 in which p is an integer from 1 to 10, such as 2, 3, 4, 5, 6, 7, 8 or 9.

10. Peptide labelled with fluorine-18 according to Claim 9, in which the compound of formula (CII) is chosen from:
- 1-[2-(2-[¹⁸F]fluoropyridin-3-yloxy)ethyl]pyrrole-2,5-dione;
- 1-[4-(2-[¹⁸F]fluoropyridin-3-yloxy)butyl]pyrrole-2,5-dione;
- 1-[5-(2-[¹⁸F]fluoropyridin-3-yloxy)pentyl]pyrrole-2,5-dione;
- 1-[6-(2-[¹⁸F]fluoropyridin-3-yloxy)hexyl]pyrrole-2,5-dione;
- 1-[(2-[¹⁸F]fluoropyridin-3-yloxy)methyl]pyrrole-2,5-dione;
- 1-[3-(2-[¹⁸F]fluoropyridin-3-yloxy)propyl]pyrrole-2,5-dione.

11. Peptide labelled with fluorine-18 according to Claim 8, in which the compound of formula (CI) corresponds to the following formula (CIII): 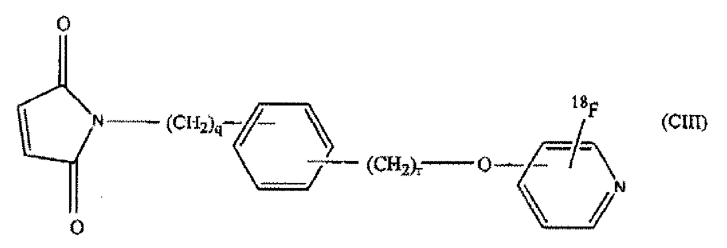 in which q and r represent independently an integer from 0 to 10, such as 0, 1, 2, 3, 4, 5, 6, 7, 8, 9.

12. Peptide labelled with fluorine-18 according to Claim 11, in which the compound of the formula (CIII) is chosen from:
- 1-{4-[2-(2-[¹⁸F]fluoropyridin-3-yloxy)ethyl]phenyl}pyrrole-2,5-dione;
- 1-[4-(2-[¹⁸F]fluoropyridin-3-yloxymethyl)phenyl]pyrrole-2,5-dione;
- 1-[4-(2-[¹⁸F]fluoropyridin-3-yloxymethyl)benzyl]pyrrole-2,5-dione.

13. Peptide labelled with fluorine-18 according to Claim 8, in which the compound of formula (CI) corresponds to the following formula (CIV):

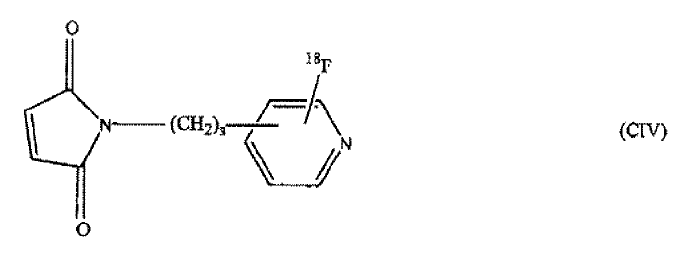

14. Peptide labelled with fluorine-18 according to Claim 13, in which the compound of formula (CIV) is 1-[3-(6-[¹⁸F]fluoropyridin-3-yl)propyl]pyrrole-2,5-dione.

15. Peptide labelled with fluorine-18 according to Claim 8, in which the compound of formula (CI) corresponds to the following formula (CV): 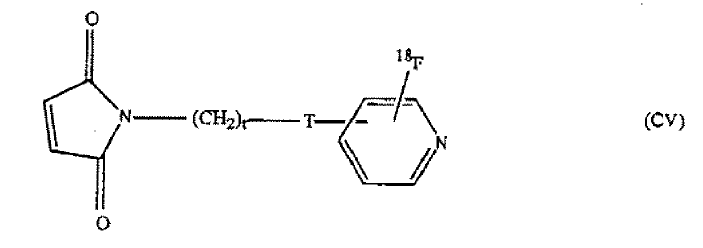 in which t is an integer from 0 to 10, such as 1, 2, 3, 4, 5, 6, 7, 8, 9 and T is a group -CH=CH- or -C≡C-.

16. Peptide labelled with fluorine-18 according to Claim 15, in which the compound (CV) is chosen from:
- 1-[3-(6-[¹⁸F]fluoropyridin-3-yl)allyl]pyrrole-2,5-dione;
- 1-[3-(6-[¹⁸F]fluoropyridin-3-yl)prop-2-ynyl]pyrrole-2,5-dione.

17. Peptide labelled with fluorine-18 according to any one of Claims 3 to 5,
in which the peptide sequence is chosen from the sequence ID No. 1, ID No. 2, ID No. 3, ID No. 4, ID No. 5, ID No. 6, ID No. 7, ID No. 8, ID No. 9, ID No. 10, ID No. 11, ID No. 12, ID No. 13 and ID No. 14 of the appended sequence listing,
in which the compound (CI) is chosen from:
- 1-[3-(6-[¹⁸F]fluoropyridin-3-yl)allyl]pyrrole-2,5-dione;
- 1-[3-(6-[¹⁸F]fluoropyridin-3-yl)prop-2-ynyl]pyrrole-2,5-dione.

18. Method for synthesizing a peptide labelled with a radioactive halogen according to any one of Claims 1 to 5, comprising a step for adding a compound (CI) of general formula: 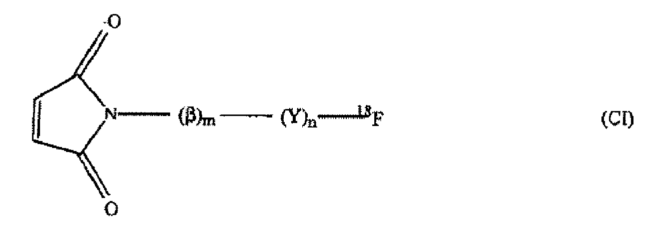 in which:
- m represents an integer from 0 to 10, such as 0, 1, 2, 3, 4, 5, 6, 7, 8, 9 or 10;
- n represents an integer from 0 to 10, such as 0, 1, 2, 3, 4, 5, 6, 7, 8, 9 or 10;
- Y represents a group chosen from alkyl groups, monocyclic or bicyclic heterocyclic groups chosen from imidazolyl, pyrazolyl, benzimidazolyl, pyridinyl, piridazinyl, pyrimidinyl, pyrazinyl, triazinyl, quinolinyl, isoquinolinyl, cinnolinyl, quinazolinyl, quinoxalinyl and purinyl groups, it being possible for Y to be optionally substituted with one or more substituents, each of these substituents being chosen independently from hydrogen, halogens, phenyl, C₁₋₆ alkyl, C₁₋₆ alkoxy, aryloxy, amino, mono- or di(C₁₋₆ alkyl) amino, mono- or di(aryl)amino, thio, C₁₋₆ alkylthio, arylthio, formyl, C₁₋₆ alkylcarbonyl, arylcarbonyl, carbonyl, C₁₋₆ alkoxycarbonyl, aryloxycarbonyl, C₁₋₆ alkylaminocarbonyl, arylaminocarbonyl and trifluoromethyl groups;
- β represents a radical of formula:
(γ)ₐ-((CR₁R₂) b- (V)_{c})_{d}- ((CR₃R₄) ₑ- (W)_{f})_{g}-
in which:
- a, b, c, d, e, f, g each independently represent an integer from 0 to 10, such as 0, 1, 2, 3, 4, 5, 6, 7, 8, 9;
- γ, V and W each independently represent -NR-₁, -0-, -S-, 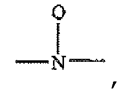 ethynyl, -CR₁=CR₂, -(C=O)-, -(C=S)-, -C (=NR₁) -, -C(=O)O-, - (C=S) S-, -C (=NR₁) NR₂-, -CR₁R₂-, -CR₁OR₂-, -CR₁NR₂R₃-, where R₁, R₂, R₃ and R₄ are independently chosen from hydrogen, halogens, phenyl, C₁₋₆ alkyl, C₁₋₆ alkoxy, aryloxy, amino, mono- or di (C₁₋₆ alkyl) amino, mono- or di(aryl)amino, thio, C₁₋₆ alkylthio, arylthio, formyl, C₁₋₆ alkylcarbonyl, arylcarbonyl, carbonyl (C₁₋₆)alkoxycarbonyl, aryloxycarbonyl, C₁₋₆ alkylaminocarbonyl, arylaminocarbonyl and trifluoromethyl groups;
directly or indirectly onto an -SH functional group of a peptide.

19. Method according to Claim 18, in which the addition is carried out directly onto a free -SH functional group of the peptide sequence (PI), for example the thiol functional group of a cystein of the peptide sequence.

20. Kit for analysis and detection of negative charges at the surface of cells, **characterized in that** it comprises a peptide labelled with fluorine-18 according to any one of Claims 1 to 17.

21. Diagnostic kit comprising a peptide labelled with fluorine-18 according to any one of Claims 1 to 17.

22. Kit for analysis and detection of microvesicles in blood, **characterized in that** it comprises a peptide labelled with fluorine-18 according to any one of Claims 1 to 17.

23. Use of a peptide labelled with fluorine-18 according to any one of Claims 1 to 17 for the manufacture of a product intended for the detection of centres exposing negatively charged lipids at the surface of cells and/or the release of microvesicles into the blood.

24. Use according to Claim 23, in which the detection is a detection by means of scintigraphic images acquired by positron emission tomography (PET).

25. Composition for analysis and detection for example by positron emission tomography (PET) having a peptide labelled with fluorine-18 according to any one of Claims 1 to 17 and a pharmaceutically acceptable vehicle.

26. Composition for diagnosis, comprising a peptide labelled with fluorine-18 according to any one of Claims 1 to 17 and a pharmaceutically acceptable vehicle.

## Patentansprüche

1. Fluor-18-markiertes Peptid, **dadurch gekennzeichnet, dass** es die folgende Peptidsequenz umfasst: worin: J, Z, U, X und B wie folgt Aminosäuren darstellen:
- die Aminosäuren J sind unabhängig voneinander aus den natürlichen Aminosäuren oder deren Derivaten auf eine solche Weise ausgewählt, dass mindestens 50 % davon aus Arg, Asn, Asp, Cys, Gln, Glu, Gly, His, Lys, Orn, Pro, Ser, Thr und Tyr ausgewählte polare Reste sind,
- die Aminosäure X¹⁸ ist unabhängig von den anderen Aminosäuren der Sequenz aus Ala, Asn, Cys, Gln, Gly, His, Ile, Leu, Met, Phe, Ser, Thr, Trp, Tyr und Val ausgewählt,
- die Aminosäuren Z⁵⁹ und Z⁶⁵ sind unabhängig aus Glu, Asp, Lys oder Arg ausgewählt,
- die Aminosäuren U und B der Sequenz sind gemäß einem der in der folgenden Tabelle 1 dargestellten Beispiele a) bis j) ausgewählt:
| | **U⁸** | **U¹¹** | **U¹⁵** | **U²⁵** | **U²⁹** | **B³⁷** | **U⁴⁰** | **U⁴⁴** | **U⁵²** | **U⁵⁶** | **U⁶⁸** | **U⁷²** |
|---|---|---|---|---|---|---|---|---|---|---|---|---|
| **Ex a)** | Val | Leu | Met | Ile | Leu | Arg | Ile | Tyr | Leu | Leu | Val | Leu |
| **Ex b)** | Ala | Ile | Ile | Ile | Leu | Arg | Ile | Tyr | Leu | Leu | Ile | Leu |
| **Ex c)** | Ala | Ile | Ile | Ile | Leu | Arg | Ile | Tyr | Leu | Leu | Met | Val |
| **Ex d)** | Ala | Leu | Met | Leu | Leu | Arg | Ile | Tyr | Leu | Leu | Ile | Met |
| **Ex e)** | Ala | Leu | Met | Ile | Ile | Arg | Val | Tyr | Leu | Leu | Ile | Met |
| **Ex f)** | Ala | Leu | Met | Ile | Ile | Arg | Ile | Phe | Leu, | Leu | Ile | Met |
| **Ex g)** | Ala | Leu | Met | Ile | Val | Arg | Ile | Phe | Leu | Leu | Ile | Phe |
| **Ex h)** | Val | Leu | Met | Ile | Leu | Arg | Ile | Phe | Leu | Leu | Ile | Met |
| **Ex i)** | Ala | Leu | Met | Ile | Leu | Arg | Ile | Phe | Leu | Leu | Ile | Met |
| **Ex j)** | Ala | Leu | Met | Ile | Leu | Arg | Ile | Tyr | Leu | Leu | Ala | Ala |
| **Ex k)** | Val | Leu | Met | Ile | Leu | Arg | Ile | Tyr | Leu | Leu | Val | Leu |
| **Ex l)** | Val | Leu | Met | Ile | Leu | Arg | Ile | Phe | Leu | Leu | Val | Leu |
| | | | | | | | | | | | | |
|---|---|---|---|---|---|---|---|---|---|---|---|---|
| (Ex = Beispiel), | | | | | | | | | | | | |
wobei die Exponenten von J, Z, U, X und B die Position dieser Aminosäuren in der Sequenz bedeuten und das Peptid direkt oder indirekt mit einer Verbindung (Cl) der allgemeinen Formel 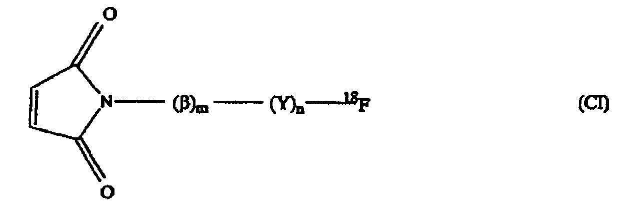 worin:
- m eine ganze Zahl von 0 bis 10 wie etwa 0, 1, 2, 3, 4, 5, 6, 7, 8, 9 oder 10 darstellt,
- n eine ganze Zahl von 0 bis 10 wie etwa 0, 1, 2, 3, 4, 5, 6, 7, 8, 9 oder 10 darstellt,
- Y eine Gruppe darstellt, die aus Alkylgruppen oder monocyclischen oder bicyclischen, heterocyclischen Gruppen ausgewählt ist, die aus Imidazolyl-, Pyrazolyl-, Benzimidazolyl-, Pyridinyl-, Pyridazinyl-, Pyrimidinyl-, Pyrazinyl-, Triazinyl-, Chinolinyl-, Isochinolinyl-, Cinnolinyl-, Chinazolinyl-, Chinoxalinyl- und Purinylgruppen ausgewählt sind, wobei Y gegebenenfalls durch einen oder mehrere Substituenten substituiert sein kann und jeder dieser Substituenten unabhängig aus Wasserstoff, Halogenen, Phenyl-, C₁₋₆-Alkyl-, C₁₋₆-Alkoxy-, Aryloxy-, Amino-, Mono- oder Di(C₁₋₆-alkyl)amino-, Mono- oder Di(aryl)amino-, Thio-, C₁₋₆-Alkylthio-, Arylthio-, Formyl-, C₁₋₆-Alkylcarbonyl-, Arylcarbonyl-, Carbonyl-, C₁₋₆-Alkoxycarbonyl-, Aryloxycarbonyl-, C₁₋₆-Alkylaminocarbonyl-, Arylaminocarbonyl- und Trifluormethylgruppen ausgewählt ist, und
- β einen Rest der Formel
(γ)ₐ-((CR₁R₂)_{b}-(V)_{c})_{d}-((CR₃R₄)ₑ-(W)_{f})_{g}-
darstellt, worin:
- a, b, c, d, e, f und g jeweils unabhängig eine ganze Zahl von 0 bis 10 wie etwa 0, 1, 2, 3, 4, 5, 6, 7, 8 oder 9 darstellen,
- γ, V und W jeweils unabhängig -NR-₁, -O-, -S-, 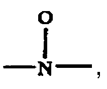 Ethinyl, -CR₁=CR₂-, -(C=O)-, -(C=S)-, -C(=NR₁)-, -C(=O)O-, -(C=S)S-, -C(=NR₁)NR₂-, -CR₁R₂-, -CR₁OR₂-, -CR₁NR₂R₃- darstellen, worin R₁, R₂, R₃ und R₄ jeweils unabhängig aus Wasserstoff, Halogenen, Phenyl-, C₁₋₆-Alkyl-, C₁₋₆-Alkoxy-, Aryloxy-, Amino-, Mono- oder Di(C₁₋₆-alkyl)amino-, Mono- oder Di(aryl)amino-, Thio-, C₁₋₆-Alkylthio-, Arylthio-, Formyl-, C₁₋₆-Alkylcarbonyl-, Arylcarbonyl-, Carbonyl-, C₁₋₆-Alkoxycarbonyl-, Aryloxycarbonyl-, C₁₋₆-Alkylaminocarbonyl-, Arylaminocarbonyl- und Trifluormethylgruppen ausgewählt sind, direkt oder indirekt an einer-SH-Funktion markiert ist.

2. Fluor-18-markiertes Peptid gemäß Anspruch 1, bei dem die Aminosäuren J unabhängig voneinander aus Ala, Arg, Asn, Asp, Cys, Gln, Glu, Gly, His, Ile, Leu, Lys, Met, Phe, Pro, Ser, Thr, Trp, Tyr und Val auf eine solche Weise ausgewählt, dass mindestens 50 % davon aus Arg, Asn, Asp, Cys, Gln, Glu, Gly, His, Lys, Pro, Ser und Thr ausgewählte polare Reste sind.

3. Fluor-18-markiertes Peptid, das eine Peptidsequenz umfasst, die aus der Sequenz ID Nr. 1, ID Nr. 2, ID Nr. 3, ID Nr. 4, ID Nr. 5, ID Nr. 6, ID Nr. 7, ID Nr. 8, ID Nr. 9, ID Nr. 10, ID Nr. 11, ID Nr. 12, ID Nr. 13 und ID Nr. 14 der angefügten Sequenzliste ausgewählt ist und bei der das Peptid direkt oder indirekt mit einer Verbindung (CI) der allgemeinen Formel 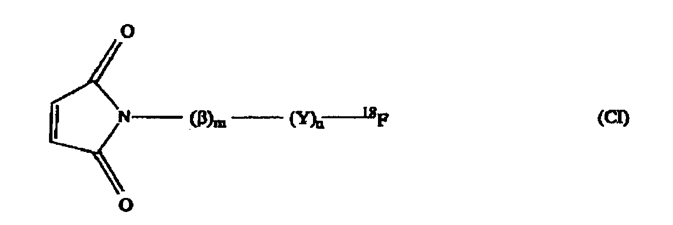 worin:
- m eine ganze Zahl von 0 bis 10 wie etwa 0, 1, 2, 3, 4, 5, 6, 7, 8, 9 oder 10 darstellt,
- n eine ganze Zahl von 0 bis 10 wie etwa 0, 1, 2, 3, 4, 5, 6, 7, 8, 9 oder 10 darstellt,
- Y eine Gruppe darstellt, die aus Alkylgruppen oder monocyclischen oder bicyclischen, heterocyclischen Gruppen ausgewählt ist, die aus Imidazolyl-, Pyrazolyl-, Benzimidazolyl-, Pyridinyl-, Pyridazinyl-, Pyrimidinyl-, Pyrazinyl-, Triazinyl-, Chinolinyl-, Isochinolinyl-, Cinnolinyl-, Chinazolinyl-, Chinoxalinyl- und Purinylgruppen ausgewählt sind, wobei Y gegebenenfalls durch einen oder mehrere Substituenten substituiert sein kann und jeder dieser Substituenten unabhängig aus Wasserstoff, Halogenen, Phenyl-, C₁₋₆-Alkyl-, C₁₋₆-Alkoxy-, Aryloxy-, Amino-, Mono- oder Di(C₁₋₆-alkyl)amino-, Mono- oder Di(aryl)amino-, Thio-, C₁₋₆-Alkylthio-, Arylthio-, Formyl-, C₁₋₆-Alkylcarbonyl-, Arylcarbonyl-, Carbonyl-, C₁₋₆-Alkoxycarbonyl-, Aryloxycarbonyl-, C₁₋₆-Alkylaminocarbonyl-, Arylaminocarbonyl- und Trifluormethylgruppen ausgewählt ist, und
- β einen Rest der Formel
(γ)ₐ-((CR₁R₂)_{b}-(V)_{c})_{d}-((CR₃R₄)ₑ-(W)_{f})_{g}-
darstellt, worin:
- a, b, c, d, e, f und g jeweils unabhängig eine ganze Zahl von 0 bis 10 wie etwa 0, 1, 2, 3, 4, 5, 6, 7, 8 oder 9 darstellen,
- γ, V und W jeweils unabhängig -NR-₁, -O-, -S-, 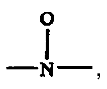 Ethinyl, -CR₁=CR₂-, -(C=O)-, -(C=S)-, -C(=NR₁)-, -C(=O)O-, -(C=S)S-, -C(=NR₁)NR₂-, -CR₁R₂-, -CR₁OR₂-, -CR₁NR₂R₃- darstellen, worin R₁, R₂, R₃ und R₄ jeweils unabhängig aus Wasserstoff, Halogenen, Phenyl-, C₁₋₆-Alkyl-, C₁₋₆-Alkoxy-, Aryloxy-, Amino-, Mono- oder Di(C₁₋₆-alkyl)amino-, Mono- oder Di(aryl)amino-, Thio-, C₁₋₆-Alkylthio-, Arylthio-, Formyl-, C₁₋₆-Alkylcarbonyl-, Arylcarbonyl-, Carbonyl-, C₁₋₆-Alkoxycarbonyl-, Aryloxycarbonyl-, C₁₋₆-Alkylaminocarbonyl-, Arylaminocarbonyl- und Trifluormethylgruppen ausgewählt sind, direkt oder indirekt an einer -SH-Funktion markiert ist.

4. Fluor-18-markiertes Peptid gemäß einem der Ansprüche 1 bis 3, das außerdem eine an sein N-terminales Ende gebundene Aminosäuresequenz umfasst, die aus Gly-Ser-Cys und Gly-Cys-Ser ausgewählt ist.

5. Fluor-18-markiertes Peptid gemäß einem der Ansprüche 1 bis 3, das außerdem eine an sein N-terminales Ende gebundene Aminosäuresequenz umfasst, die aus Gly-Ser-Gly-Cys, Gly-Cys-Gly-Ser und Gly-Cys-Gly-Cys ausgewählt ist.

6. Fluor-18-markiertes Peptid gemäß einem der Ansprüche 1 bis 5, bei dem das Peptid mit der Verbindung (CI) durch Kuppeln der Maleimidfunktion der Verbindung (CI) mit einer freien -SH-Funktion des Peptids, zum Beispiel der Thiolfunktion eines Cysteins des Peptids direkt markiert ist.

7. Fluor-18-markiertes Peptid gemäß einem der Ansprüche 1 bis 5, bei dem das Peptid mit der Verbindung (CI) durch Kuppeln der Maleimidfunktion der Verbindung (Cl) mit einer freien -SH-Funktion der Peptidsequenz (Pl), zum Beispiel der Thiolfunktion eines Cysteins der Peptidsequenz direkt markiert ist.

8. Fluor-18-markiertes Peptid gemäß einem der Ansprüche 1 bis 5, bei dem bei der Verbindung der Formel (CI) n = 1 und Y eine 3-Pyridinylgruppe ist.

9. Fluor-18-markiertes Peptid gemäß Anspruch 8, bei dem die Verbindung (Cl) der folgenden Formel (CII) entspricht: 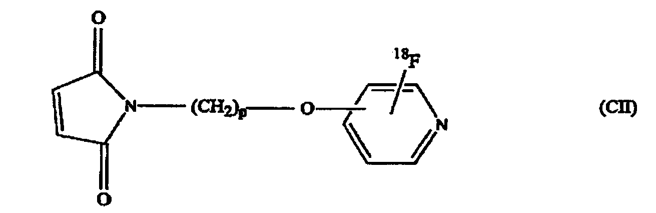 worin p eine ganze Zahl von 1 bis 10 wie etwa 2, 3, 4, 5, 6, 7, 8 oder 9 darstellt.

10. Fluor-18-markiertes Peptid gemäß Anspruch 9, bei dem die Verbindung der Formel (CII) aus
- 1-[2-(2-[¹⁸F]Fluorpyridin-3-yloxy)ethyl]pyrrol-2,5-dion,
- 1-[4-(2-[¹⁸F]Fluorpyridin-3-yloxy)butyl]pyrrol-2,5-dion,
- 1-[5-(2-[¹⁸F]Fluorpyridin-3-yloxy)pentyl]pyrrol-2,5-dion,
- 1-[6-(2-[¹⁸F]Fluorpyridin-3-yloxy)hexyl]pyrrol-2,5-dion,
- 1-[(2-[¹⁸F]Fluorpyridin-3-yloxy)methyl]pyrrol-2,5-dion,
- 1-[3-(2-[¹⁸F]Fluorpyridin-3-yloxy)propyl]pyrrol-2,5-dion, ausgewählt ist.

11. Fluor-18-markiertes Peptid gemäß Anspruch 8, bei dem die Verbindung der Formel (Cl) der folgenden Formel (CIII) entspricht: 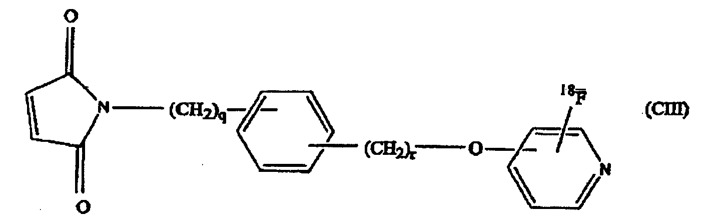 worin q und r unabhängig eine ganze Zahl von 0 bis 10 wie etwa 0, 1, 2, 3, 4, 5, 6, 7, 8 oder 9 darstellen.

12. Fluor-18-markiertes Peptid gemäß Anspruch 11, bei dem die Verbindung der Formel (CIII) aus
- 1-{4-[2-(2-[¹⁸F]Fluorpyridin-3-yloxy)ethyl]phenyl}pyrrol-2,5-dion,
- 1-[4-(2--[¹⁸F]Fluorpyridin-3-yloxymethyl)phenyl]pyrrol-2,5-dion,
- 1-[4-(2--[¹⁸F]Fluorpyridin-3-yloxymethyl)benzyl]pyrrol-2,5-dion ausgewählt ist.

13. Fluor-18-markiertes Peptid gemäß Anspruch 8, bei dem die Verbindung der Formel (Cl) der folgenden Formel (CIV) entspricht:

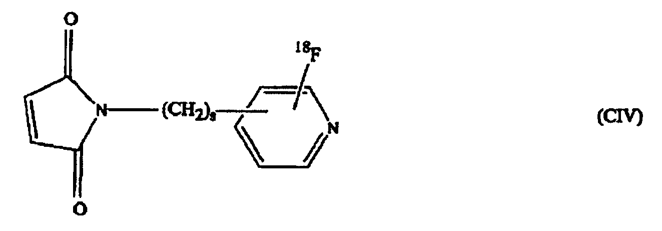

14. Fluor-18-markiertes Peptid gemäß Anspruch 13, bei dem die Verbindung der Formel (CIV) 1-[3-(6-[¹⁸F]Fluorpyridin-3-yl)propyl]pyrrol-2,5-dion ist.

15. Fluor-18 markiertes Peptid gemäß Anspruch 8, bei dem die Verbindung der Formel (Cl) der folgenden Formel (CV) entspricht: 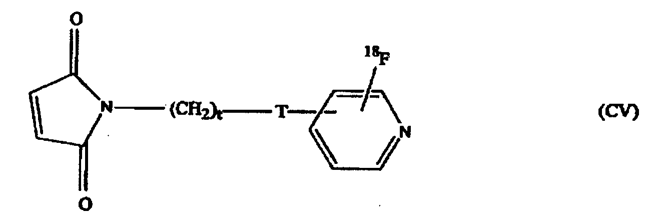 worin t eine Zahl von 0 bis 10 wie etwa 1, 2, 3, 4, 5, 6, 7, 8 oder 9 darstellt und T eine Gruppe -CH=CH- oder -C=C- ist.

16. Fluor-18-markiertes Peptid gemäß Anspruch 15, bei dem die Verbindung (CV) aus
- 1-[3-(6-[¹⁸F]Fluorpyridin-3-yl)allyl]pyrrol-2,5-dion und
- 1-[3-(6-[¹⁸F]Fluorpyridin-3-yl)prop-2-inyl]pyrrol-2,5-dion ausgewählt ist.

17. Fluor-18-markiertes Peptid gemäß einem der Ansprüche 3 bis 5, bei dem die Peptidsequenz aus der Sequenz ID Nr. 1, ID Nr. 2, ID Nr. 3, ID Nr. 4, ID Nr. 5, ID Nr. 6, ID Nr. 7, ID Nr. 8, ID Nr. 9, ID Nr. 10, ID Nr. 11, ID Nr. 12, ID Nr. 13 und ID Nr. 14 der angefügten Sequenzliste ausgewählt ist und die Verbindung (Cl) aus
- 1-[3-(6-[¹⁸F]Fluorpyridin-3-yl)allyl]pyrrol-2,5-dion und
- 1-[3-(6-[¹⁸F]Fluorpyridin-3-yl)prop-2-inyl]pyrrol-2,5-dion ausgewählt ist.

18. Verfahren zur Synthese eines durch ein radioaktives Halogen substituierten Peptids gemäß einem der Ansprüche 1 bis 5, das einen Schritt der direkten oder indirekten Addition einer Verbindung (Cl) der allgemeinen Formel 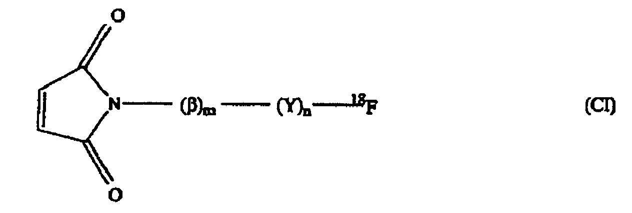 worin:
- m eine ganze Zahl von 0 bis 10 wie etwa 0, 1, 2, 3, 4, 5, 6, 7, 8, 9 oder 10 darstellt,
- n eine ganze Zahl von 0 bis 10 wie etwa 0, 1, 2, 3, 4, 5, 6, 7, 8, 9 oder 10 darstellt,
- Y eine Gruppe darstellt, die aus Alkylgruppen oder monocyclischen oder bicyclischen, heterocyclischen Gruppen ausgewählt ist, die aus Imidazolyl-, Pyrazolyl-, Benzimidazolyl-, Pyridinyl-, Pyridazinyl-, Pyrimidinyl-, Pyrazinyl-, Triazinyl-, Chinolinyl-, Isochinolinyl-, Cinnolinyl-, Chinazolinyl-, Chinoxalinyl- und Purinylgruppen ausgewählt sind, wobei Y gegebenenfalls durch einen oder mehrere Substituenten substituiert sein kann und jeder dieser Substituenten unabhängig aus Wasserstoff, Halogenen, Phenyl-, C₁₋₆-Alkyl-, C₁₋₆-Alkoxy-, Aryloxy-, Amino-, Mono- oder Di(C₁₋₆-alkyl)amino-, Mono- oder Di(aryl)amino-, Thio-, C₁₋₆-Alkylthio-, Arylthio-, Formyl-, C₁₋₆-Alkylcarbonyl-, Arylcarbonyl-, Carbonyl-, C₁₋₆-Alkoxycarbonyl-, Aryloxycarbonyl-, C₁₋₆-Alkylaminocarbonyl-, Arylaminocarbonyl- und Trifluormethylgruppen ausgewählt ist, und
- β einen Rest der Formel
(γ)ₐ-((CR₁R₂)_{b}-(V)_{c})_{d}-((CR₃R₄)ₑ-(W)_{f})_{g}-
darstellt, worin:
- a, b, c, d, e, f und g jeweils unabhängig eine ganze Zahl von 0 bis 10 wie etwa 0, 1, 2, 3, 4, 5, 6, 7, 8 oder 9 darstellen,
- γ, V und W jeweils unabhängig -NR-₁, -O-, -S-, 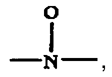 Ethinyl, -CR₁=CR₂-, -(C=O)-, -(C=S)-, -C(=NR₁)-, -C(=O)O-, -(C=S)S-, -C(=NR₁)NR₂-, -CR₁R₂-, -CR₁OR₂-, -CR₁NR₂R₃- darstellen, worin R₁, R₂, R₃ und R₄ jeweils unabhängig aus Wasserstoff, Halogenen, Phenyl-, C₁₋₆-Alkyl-, C₁₋₆-Alkoxy-, Aryloxy-, Amino-, Mono- oder Di(C₁₋₆-alkyl)amino-, Mono- oder Di(aryl)amino-, Thio-, C₁₋₆-Alkylthio-, Arylthio-, Formyl-, C₁₋₆-Alkylcarbonyl-, Arylcarbonyl-, Carbonyl-, C₁₋₆-Alkoxycarbonyl-, Aryloxycarbonyl-, C₁₋₆-Alkylaminocarbonyl-, Arylaminocarbonyl- und Trifluormethylgruppen ausgewählt sind,
direkt oder indirekt an eine -SH-Funktion eines Peptids umfasst.

19. Verfahren gemäß Anspruch 18, wobei die Addition direkt an einer freien -SH-Funktion der Peptidsequenz (PI), zum Beispiel der Thiolfunktion eines Cysteins der Peptidsequenz ausgeführt wird.

20. Kit zur Analyse und zum Nachweis negativer Ladungen auf der Oberfläche von Zellen, **dadurch gekennzeichnet, dass** er ein Fluor-18-markiertes Peptid gemäß einem der Ansprüche 1 bis 17 umfasst.

21. Diagnostischer Kit umfassend ein Fluor-18-markiertes Peptid gemäß einem der Ansprüche 1 bis 17.

22. Kit zur Analyse und zum Nachweis von Mikrovesikeln im Blut, **dadurch gekennzeichnet, dass** er ein Fluor-18-markiertes Peptid gemäß einem der Ansprüche 1 bis 17 umfasst.

23. Verwendung eines Fluor-18-markierten Peptids gemäß einem der Ansprüche 1 bis 17 zur Herstellung eines Produkts, das zum Nachweis von Zentren, die negativ geladene Lipide an der Zelloberfläche exponieren, und/oder der Freisetzung von Mikrovesikeln in das Blut bestimmt ist

24. Verwendung gemäß Anspruch 23, wobei der Nachweis ein Nachweis mittels szintigraphischer Bilder ist, die durch Positronenemissionstomographie (PET) aufgenommen wurden.

25. Zusammensetzung zur Analyse und zum Nachweis, zum Beispiel durch Positronenemissionstomographie (PET), die ein Fluor-18-markiertes Peptid gemäß einem der Ansprüche 1 bis 17 und einen pharmazeutisch annehmbaren Träger aufweist.

26. Zusammensetzung zur Diagnostik, die ein Fluor-18-markiertes Peptid gemäß einem der Ansprüche 1 bis 17 und einen pharmazeutisch annehmbaren Träger umfasst.


### LISTE DE SEQUENCES

<110> COMMISSARIAT A L'ENERGIE ATOMIQUE UNIVERSITE PIERRE ET MARIE CURIE (PARIS VI)
<120> PEPTIDES MARQUE AYANT UNE AFFINITE POUR UN PHOSPHOLIPIDE ET UTILISATIONS
<130> B14023EE
<140>
   <141>
<150> FR N°02 08204
   <151> 2002-07-01
<160> 14
<170> PatentIn Ver. 2.1
<210> 1
   <211> 75
   <212> PRT
   <213> Séquence artificielle
<220>
   <223> Description de la séquence artificielle: séquence dérivée d'une annexine humaine
<400> 1
   Gly Phe Asp Glu Arg Ala Asp Val Glu Thr Leu Arg Lys Ala Met Lys
    1               5                  10                  15
   Gly Leu Gly Thr Asp Glu Glu Ser Ile Leu Thr Leu Leu Thr Ser Arg
               20                  25                  30
   Ser Asn Ala Gln Arg Gln Glu Ile Ser Ala Ala Tyr Lys Thr Leu Phe
           35                  40                  45
   Gly Arg Asp Leu Leu Asp Asp Leu Lys Ser Glu Leu Thr Gly Lys Phe
       50                  55                  60
   Glu Lys Leu Val Val Ala Leu Leu Lys Pro Ser
   65                  70                  75
<210> 2
   <211> 75
   <212> PRT
   <213> Séquence artificielle
<220>
   <223> Description de la séquence artificielle: séquence dérivée d'une annexine humaine
<400> 2
   Asn Phe Asp Ala Glu Arg Asp Ala Leu Asn Ile Arg Lys Ala Ile Lys
   1 5 10 15
   Gly Met Gly Val Asp Glu Asp Thr Ile Val Asn Ile Leu Thr Asn Arg
   20 25 30
   Ser Asn Ala Gln Arg Gln Asp Ile Ala Phe Ala Tyr Gln Arg Arg Thr
   35 40 45
   Lys Arg Glu Leu Ala Ser Asp Leu Lys Ser Glu Leu Ser Gly His Leu
   50 55 60
   Glu Arg Val Ile Leu Gly Leu Leu Lys Thr Ser
   65 70 75
<210> 3
   <211> 75
   <212> PRT
   <213> Séquence artificielle
<220>
   <223> Description de la séquence artificielle: séquence dérivée d'une annexine humaine
<400> 3
   Asp Phe Ser Pro Ser Val Asp Ala Glu Ala Ile Arg Lys Ala Ile Lys
   1 5 10 15
   Gly Ile Gly Thr Asp Glu Asp Met Leu Ile Ser Ile Leu Thr Glu Arg
   20 25 30
   Ser Asn Ala Gln Arg Gln Leu Ile Val Lys Glu Tyr Gln Ala Ala Tyr
   35 40 45
   Gly Arg Glu Leu Lys Asp Asp Leu Lys Ser Glu Leu Ser Gly His Phe
   50 55 60
   Glu Arg Leu Met Val Ala Leu Val Thr Pro Ser
   65 70 75
<210> 4
   <211> 75
   <212> PRT
   <213> Séquence artificielle
<220>
   <223> Description de la séquence artificielle: séquence dérivée d'une annexine humaine
<400> 4
   Gly Phe Asn Ala Met Glu Asp Ala Gln Thr Leu Arg Lys Ala Met Lys
   1 5 10 15
   Gly Leu Gly Thr Asp Glu Asp Ala Ile Ile Ser Val Leu Ala Tyr Arg
   20 25 30
   Asn Thr Ala Gln Arg Gln Glu Ile Arg Thr Ala Tyr Lys Ser Thr Ile
   35 40 45
   Gly Arg Asp Leu Ile Asp Asp Leu Lys Ser Glu Leu Ser Gly Asn Phe
   50 55 60
   Glu Arg Val Ile Val Gly Met Met Thr Pro Ser
   65 70 75
<210> 5
   <211> 75
   <212> PRT
   <213> Séquence artificielle
<220>
   <223> Description de la séquence artificielle: séquence dérivée d'une annexine humaine
<400> 5
   Gly Phe Asp Pro Asn Gln Asp Ala Glu Ala Leu Arg Thr Ala Met Lys
   1 5 10 15
   Gly Phe Gly Ser Asp Glu Glu Ala Ile Leu Asp Ile Ile Thr Ser Arg
   20 25 30
   Ser Asn Arg Gln Arg Gln Glu Val Cys Gln Ser Tyr Lys Ser Leu Tyr
   35 40 45
   Gly Arg Asp Leu Ile Ala Asp Leu Lys Ser Glu Leu Thr Gly Lys Phe
   50 55 60
   Glu Arg Leu Ile Val Gly Leu Met Arg Pro Ser
   65 70 75
<210> 6
   <211> 75
   <212> PRT
   <213> Séquence artificielle
<220>
   <223> Description de la séquence artificielle: séquence dérivée d'une annexine humaine
<400> 6
   Gly Phe Asn Pro Asp Ala Asp Ala Lys Ala Leu Arg Lys Ala Met Lys
   1 5 10 15
   Gly Leu Gly Thr Asp Glu Asp Thr Ile Ile Asp Ile Ile Thr His Arg
   20 25 30
   Ser Asn Val Gln Arg Gln Gln Ile Arg Gln Thr Phe Lys Ser His Phe
   35 40 45
   Gly Arg Asp Leu Met Thr Asp Leu Lys Ser Glu Ile Ser Gly Asp Leu
   50 55 60
   Glu Arg Leu Ile Leu Gly Leu Met Met Pro Ser
   65 70 75
<210> 7
   <211> 75
   <212> PRT
   <213> Séquence artificielle
<220>
   <223> Description de la séquence artificielle: séquence dérivée d'une annexine humaine
<400> 7
   Pro Gly Asp Ala Ile Arg Asp Ala Glu Ile Leu Arg Lys Ala Met Lys
   1 5 10 15
   Gly Phe Gly Thr Asp Glu Gln Ala Ile Val Asp Val Val Ala Asn Arg
   20 25 30
   Ser Asn Asp Gln Arg Gln Lys Ile Lys Ala Ala Phe Lys Thr Ser Tyr
   35 40 45
   Gly Arg Asp Leu Ile Lys Asp Leu Lys Ser Glu Leu Ser Gly Asn Met
   50 55 60
   Glu Arg Leu Ile Leu Ala Leu Phe Met Pro Ser
   65 70 75
<210> 8
   <211> 75
   <212> PRT
   <213> Séquence artificielle
<220> .
   <223> Description de la séquence artificielle: séquence dérivée d'une annexine humaine
<400> 8
   His Phe Asn Pro Asp Pro Asp Val Glu Thr Leu Arg Lys Ala Met Lys
   1 5 10 15
   Gly Ile Gly Thr Asn Glu Gln Ala Ile Ile Asp Val Leu Thr Lys Arg
   20 25 30
   Ser Asn Thr Gln Arg Gln Thr Ile Ala Lys Ser Phe Lys Ala Gln Phe
   35 40 45
   Gly Arg Asp Leu Thr Glu Asp Leu Lys Ser Glu Leu Ser Gly Lys Leu
   50 55 60
   Glu Arg Leu Ile Val Ala Leu Met Tyr Pro Ser
   65 70 75
<210> 9
   <211> 75
   <212> PRT
   <213> Séquence artificielle
<220>
   <223> Description de la séquence artificielle: séquence dérivée d'une annexine humaine
<400> 9
   Gly Phe Asp Pro Leu Arg Asp Ala Glu Val Leu Arg Lys Ala Met Lys
   1 5 10 15
   Gly Phe Gly Thr Asp Glu Gln Ala Ile Ile Asp Cys Leu Gly Ser Arg
   20 25 30
   Ser Asn Lys Gln Arg Gln Gln Ile Leu Leu Ser Phe Lys Thr Ala Tyr
   35 40 45
   Gly Arg Asp Leu Ile Lys Asp Leu Lys Ser Glu Leu Ser Gly Asn Phe
   50 55 60
   Glu Lys Thr Ile Leu Ala Leu Met Lys Thr Ser
   65 70 75
<210> 10
   <211> 75
   <212> PRT
   <213> Séquence artificielle
<220>
   <223> Description de la séquence artificielle: séquence dérivée d'une annexine humaine
<400> 10
   Gly Phe Asp Val Asp Arg Asp Ala Lys Lys Leu Arg Lys Ala Met Lys
   1 5 10 15
   Gly Met Gly Thr Asn Glu Ala Ala Ile Ile Glu Ile Leu Ser Gly Arg
   20 25 30
   Thr Ser Asp Glu Arg Gln Gln Ile Lys Gln Lys Tyr Lys Ala Thr Tyr
   35 40 45
   Gly Arg Glu Leu Glu Glu Asp Leu Lys Ser Glu Leu Ser Gly Asn Phe
   50 55 60
   Glu Lys Thr Ala Leu Ala Leu Leu Asp Arg Ser
   65 70 75
<210> 11
   <211> 79
   <212> PRT
   <213> Séquence artificielle
<220>
   <223> Description de la séquence artificielle: séquence dérivée d'une annexine humaine
<220>
   <223> Xaa en position 22 est Leu, Met ou Trp
<220>
   <223> Xaa en position 34 est Thr ou Lys
<220>
   <223> Xaa en position 45 est Ser ou Lys
<220>
   <223> Xaa en position 48 est Phe ou Tyr
<220>
   <223> Xaa en position 50 est Thr ou Glu
<220>
   <223> Xaa en position 63 est Glu ou Lys
<220>
   <223> Xaa en position 69 est Glu ou Lys
<220>
   <223> Xaa en position 71 est Glu ou Leu
<400> 11
   Gly Ser Gly Cys Gly Phe Asp Glu Arg Ala Asp Val Glu Thr Leu Arg
     1               5                  10                  15
   Lys Ala Met Lys Gly Xaa Gly Thr Asp Glu Glu Ser Ile Leu Thr Leu
                20                  25                  30
   Leu Xaa Ser Arg Ser Asn Ala Gln Arg Gln Glu Ile Xaa Ala Ala Xaa
            35                  40                  45
   Lys Xaa Leu Phe Gly Arg Asp Leu Leu Asp Asp Leu Lys Ser Xaa Leu
        50                  55                  60
   Thr Gly Lys Phe Xaa Lys Xaa Val Val Ala Leu Leu Lys Pro Ser
    65                  70                  75
<210> 12
   <211> 78
   <212> PRT
   <213> Séquence artificielle
<220>
   <223> Description de la séquence artificielle: séquence dérivée d'une annexine humaine
<400> 12
   Gly Ser Pro Gly Phe Asp Glu Arg Ala Asp Val Glu Thr Leu Arg Lys
     1               5                  10                  15
   Ala Met Lys Gly Leu Gly Thr Asp Glu Glu Ser Ile Leu Thr Leu Leu
                20                  25                  30
   Thr Ser Arg Ser Asn Ala Gln Arg Gln Glu Ile Ser Ala Ala Tyr Lys
            35                  40                  45
   Thr Leu Phe Gly Arg Asp Leu Leu Asp Asp Leu Lys Ser Glu Leu Thr
        50                  55                  60
   Gly Lys Phe Glu Lys Leu Val Val Ala Leu Leu Lys Pro Ser
    65                  70                  75
<210> 13
   <211> 83
   <212> PRT
   <213> Séquence artificielle
<220>
   <223> Description de la séquence artificielle: séquence dérivée d'une annexine humaine
<220>
   <223> Xaa en position 25 est Leu, Met ou Trp
<220>
   <223> Xaa en position 37 est Thr ou Lys
<220>
   <223> Xaa en position 48 est Ser ou Lys
<220>
   <223> Xaa en position 51 est Phe ou Tyr
<220>
   <223> Xaa en position 53 est Thr ou Glu
<220>
   <223> Xaa en position 66 est Glu ou Lys
<220>
   <223> Xaa en position 72 est Glu ou Lys
<220>
   <223> Xaa en position 74 est Glu ou Leu
<400> 13
<210> 14
   <211> 87
   <212> PRT
   <213> Séquence artificielle
<220>
   <223> Description de la séquence artificielle: séquence dérivée d'une annexine humaine
<220>
   <223> Xaa en position 29 est Leu, Met ou Trp
<220>
   <223> Xaa en position 41 est Tyr ou Lys,
<220>
   <223> Xaa en position 52 est Ser ou Lys
<220>
   <223> Xaa en position 55 est Phe ou Tyr
<220>
   <223> Xaa en position 57 est Thr ou Glu
<220>
   <223> Xaa en position 70 est Glu ou Lys
<220>
   <223> Xaa en position 76 est Glu ou Lys
<220>
   <223> Xaa en position 78 est Glu ou Leu
<400> 14
   Gly Ser Gly Cys Gly Thr Glu Thr Asp Phe Pro Gly Phe Asp Glu Arg
    1               5                   10                  15
   Ala Asp Val Glu Thr Leu Arg Lys Ala Met Lys Gly Xaa Gly Thr Asp
               20                  25                  30
   Glu Glu Ser Ile Leu Thr Leu Leu Xaa Ser Arg Ser Asn Ala Gln Arg
           35                  40                  45
   Gln Glu Ile Xaa Ala Ala Xaa Lys Xaa Leu Phe Gly Arg Asp Leu Leu
       50                  55                  60
   Asp Asp Leu Lys Ser Xaa Leu Thr Gly Lys Phe Xaa Lys Xaa Val Val
   65                  70                  75                  80
   Ala Leu Leu Lys Pro Ser Arg
                   85